(19) Europäisches Patentamt
European Patent Office
Office européen des brevets

(11) **EP 4 578 453 A1**

(12) **EUROPEAN PATENT APPLICATION**
published in accordance with Art. 153(4) EPC

(43) Date of publication:
**02.07.2025 Bulletin 2025/27**

(21) Application number: **22955931.5**

(22) Date of filing: **22.08.2022**

(51) International Patent Classification (IPC):
***A61K 31/785*** *(2006.01)* ***C08F 226/02*** *(2006.01)*

(52) Cooperative Patent Classification (CPC):
**A61K 31/785; C08F 226/02**

(86) International application number:
**PCT/CN2022/113905**

(87) International publication number:
**WO 2024/040380 (29.02.2024 Gazette 2024/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Waterstone Pharmaceuticals (Wuhan) Co., Ltd.**
**Wuhan, Hubei 430075 (CN)**

(72) Inventors:
- **ZHOU, Youcheng**
**Wuhan, Hubei 430075 (CN)**
- **LI, Tongtong**
**Wuhan, Hubei 430075 (CN)**
- **HU, Minglong**
**Wuhan, Hubei 430075 (CN)**
- **YU, Yao**
**Wuhan, Hubei 430075 (CN)**
- **WANG, Xiaolong**
**Wuhan, Hubei 430075 (CN)**
- **ZHAO, Along**
**Wuhan, Hubei 430075 (CN)**
- **ZHANG, Faming**
**Wuhan, Hubei 430075 (CN)**

(74) Representative: **Nederlandsch Octrooibureau**
**P.O. Box 29720**
**2502 LS The Hague (NL)**

(54) **AMMONIUM SALT POLYMER, METHOD FOR PREPARING SAME, AND USE THEREOF AS BILE ACID CHELATING AGENT**

(57) An ammonium salt polymer, a method for preparing same, and use thereof as a bile acid chelating agent. The ammonium salt polymer is obtained by polymerizing a monomer of Formula (I) and a monomer of Formula (II), wherein m, n, p, q, and r are each independently selected from 1, 2 or 3. The ammonium salt polymer contains monovalent, divalent, or trivalent anions as counter ions and can be used for treating or preventing dyslipidemia (such as hyperlipidemia), cholestasis, bile acid diarrhea, and other diseases.

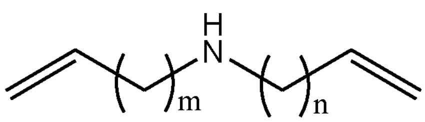

(I)

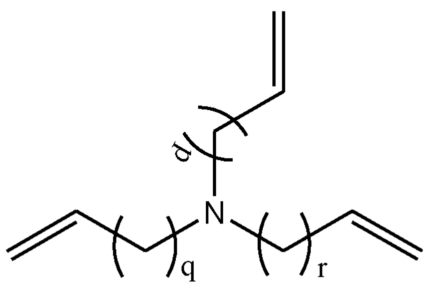

(II)

EP 4 578 453 A1

# Description

## Technical Field

The present invention belongs to the field of polymer drugs. Specifically, the present invention relates to ammonium salt polymers, methods for preparing the same, and their use as bile acid sequestrants. Said ammonium salt polymers are useful in the treatment or prevention of diseases such as dyslipidemia (for example, hyperlipidemia), cholestatic liver diseases and bile acid diarrhea (BAD).

## Background of the Invention

Bile acids are important components of bile. The physiological functions of bile acids mainly include promoting the digestion and absorption of lipids, inhibiting the precipitation of cholesterol in bile (gallstones formation), etc. Bile acids are also involved in the metabolism of glucose and lipids. Therefore, bile acids are associated with a variety of diseases, for example, dyslipidemia (for example, hyperlipidemia), cholestatic liver diseases, bile acid diarrhea, etc.

### 1. Dyslipidemia

Dyslipidemia refers to abnormal metabolism of lipoproteins in the human body. A small number of dyslipidemias are secondary dyslipidemias caused by systemic diseases, while the vast majority of dyslipidemias are primary dyslipidemias caused by inherited genetic defects or interactions thereof with environmental factors. The incidence of dyslipidemia is high in China and is gradually increasing, which is closely associated with the significant improvement in the living standards of the Chinese people and the changes in their diet habits.

Hyperlipidemia is the most common form of dyslipidemia, and also known as hyperlipoproteinemia. Hyperlipidemia refers to a condition involving abnormally elevated levels of any one or all of lipids and/or lipoproteins in the blood, including elevated total cholesterol (TC), elevated triglycerides (TG), elevated low-density lipoprotein cholesterol (LDL-c), or decreased high-density lipoprotein cholesterol (HDL-c). Clinically, plasma cholesterol higher than 230mg/100ml and triglyceride higher than 140mg/100ml are collectively referred to as hyperlipidemia.

The damage of hyperlipidemia to the human body is insidious, progressive and systemic, and it brings many problems. When the level of blood lipids is elevated for a long time, blood lipids and their decomposition products will gradually deposit on the vascular walls, accompanied by the formation of fibrous tissue, narrowing the blood vessel channels and reducing blood vessel elasticity, and eventually leading to the blockage of blood vessel. If lipid plaque is deposited on the coronary arteries, it may cause ischemic symptoms in the heart and induce coronary heart disease; if lipid plaque is deposited in the brain, it may induce cerebral infarction; if lipid plaque is deposited on the lower limbs, it may cause pain and even necrosis of the lower limbs; if lipid plaque is deposited in the orbital plexus, it may cause blindness; if lipid plaque is deposited in the liver, it may cause fatty liver. Hyperlipidemia is recognized as a risk factor for stroke, coronary heart disease, myocardial infarction, and sudden death. In addition, hyperlipidemia can also induce acute pancreatitis.

Given the adverse consequences of hyperlipidemia, administering antilipemic agents to patients is necessary. The mechanisms of action of such agents mainly include inhibiting the biosynthesis of cholesterol, reducing the level of cholesterol in the body, increasing the level of HDL, inhibiting the absorption of cholesterol, etc. For example, statins (e.g., lovastatin, simvastatin, atorvastatin, rosuvastatin) may inhibit hydroxymethylglutaryl-CoA reductase, which is the rate-limiting enzyme in the biosynthesis of cholesterol in the body, and thus reduce the biosynthesis of cholesterol in the body; phenoxyacetic acid antilipemic agents (e.g., clofibrate, fenofibrate) also interfere with the synthesis of cholesterol in the body, and significantly reduce the levels of triglycerides and total cholesterol; nicotinic acid analogs (e.g., acipimox) may increase the level of HDL; bile acid binding resins (e.g., cholestyramine, Colesevelam hydrochloride) may reduce the level of cholesterol in the body.

Among the above-mentioned antilipemic agents, bile acid binding resins are safe oral drugs with good hypolipidemic effect. Bile acids are important components of bile and play an important role in lipid metabolism. During the digestion in the body, cholesterol is the only precursor of bile acids. The bile acids produced by the decomposition of cholesterol are secreted into the intestine, where a large amount of bile acids is absorbed and then re-enter the liver through enterohepatic circulation. Bile acid binding resins are polymers that cannot be absorbed by the human body, and have great capacity for the adsorption of bile acids, thereby excreting bile acids from the body and preventing the reabsorption of bile acids. The high consumption of bile acids promotes the conversion of more cholesterol into bile acids, thereby achieving the purpose of reducing cholesterol in the body.

Colesevelam hydrochloride is the latest representative of such type of agents, has the chemical name N,N,N-trimethyl-6-(2-allylamino)-1-hexylamine copolymer hydrochloride and the following chemical structure:

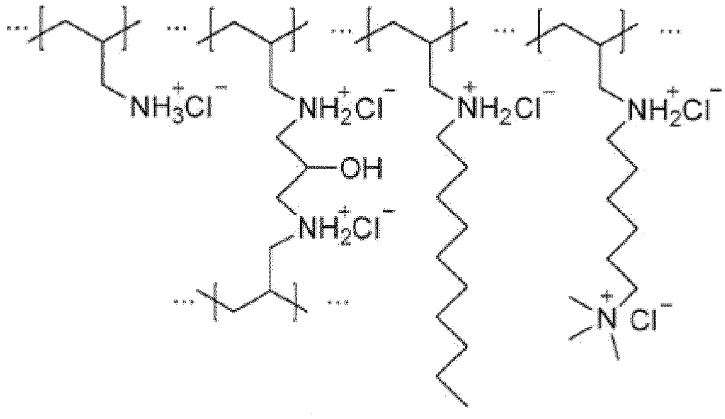

Colesevelam hydrochloride not only reduces the level of cholesterol in the body, but also increases the clearance of low-density lipoproteins from the blood. Colesevelam hydrochloride, with trade name WELCHOL, is approved as a bile acid sequestrant for the treatment of primary hyperlipidemia and type 2 diabetes.

Aging is an important challenge that many countries are facing today. Accordingly, the number of patients with hyperlipidemia is increasing continuously, and more hypolipidemic drugs are needed.

### 2. Cholestatic liver diseases

Cholestasis refers to a pathological state in which various reasons both inside and outside the liver lead to the impaired formation, secretion and excretion of bile so that bile fails to flow normally into the duodenum and instead into the blood. Clinical manifestations may include pruritus, fatigue, darker urine and jaundice, etc. Cholestasis is often asymptomatic in the early stage and only characterized by elevated serum ALP and GGT levels. Hyperbilirubinemia may occur after the progression of the disease, and liver failure or even death may occur in severe cases.

Hepatobiliary diseases with cholestasis as the main manifestation caused by various reasons are often collectively referred to as cholestatic liver diseases. The diagnostic criteria for cholestatic liver diseases usually follow the Clinical Practice Guidelines on the management of cholestatic liver diseases developed by European Association for the Study of the Liver (EASL) in 2009, that is, "ALP exceeds 1.5×ULN, and GGTexceeds 3×ULN". However, it is noted that GGT may not be elevated in some special cholestatic liver diseases such as PFIC type 1 or 2 and BRIC.

Cholestatic liver diseases can be divided into hereditary cholestatic liver diseases and acquired cholestatic liver diseases. Hereditary cholestatic liver diseases include progressive familial intrahepatic cholestasis (PFIC), benign recurrent intrahepatic cholestasis (BRIC), inborn errors of bile acid synthesis, Alagille syndrome (ALGS), neonatal cholestasis caused by Cirtin deficiency, ARC syndrome, etc. Acquired cholestatic liver diseases is often caused by infection, drugs, hormones, pregnancy, tumors, biliary occlusion and other factors, and include primary biliary cholangitis (PBC), primary sclerosing cholangitis (PSC), autoimmune hepatitis (AIH), drug-induced cholestasis (DRIC), biliary atresia (BA) and intrahepatic cholestasis of pregnancy (ICP), etc.

Currently, the main drug used to treat cholestatic liver diseases is ursodeoxycholic acid (UDCA), which has been proven to reduce cholestasis in various cholestatic diseases and is the standard first-line drug for the treatment of PBC. However, UDCA is ineffective against most pruritus and is effective in only about 50% of PBC patients, with nearly 40% of PBC patients having an inadequate response to UDCA. In addition, 5% to 10% of PBC patients are intolerant to UDCA. For patients with PBC who have an inadequate response or intolerance to UDCA, only obeticholic acid (OCA) is available. Obeticholic acid is a farnesoid X receptor (FXR) agonist, and has side effects such as severe skin pruritus, fatigue, abdominal pain and discomfort, and rash. Therefore, the use of obeticholic acid is also limited.

Maralixibat, an Apical sodium-dependent bile acid transporter (ASBT) inhibitor, is approved for the treatment of pruritus in 1 year and older pediatric patients with ALGS, but has adverse effects such as diarrhea, abdominal pain, vomiting, fat-soluble vitamin deficiency, liver test abnormalities, gastrointestinal bleeding and fractures. Odevixibat, an ileal bile acid transporter (IBAT) inhibitor, is approved for the treatment of progressive familial intrahepatic cholestasis (PFIC), and has adverse effects such as liver test abnormalities, diarrhea, abdominal pain, vomiting, and fat-soluble vitamin deficiency.

As the incidence of cholestatic liver diseases has increased significantly in recent years, there is an urgent need for novel and effective therapeutic drugs. Currently, the peroxisome proliferator-activated receptor (PPAR) agonists Elafibranor (GFT505) and Seladelpar (MBX-8025) are tested in clinical trials and are promising to be a novel drug for the clinical treatment of cholestatic liver diseases. However, preliminary results of the clinical trial show that these drugs also have serious side effects.

In contrast, bile acid binding resins are not absorbed by the intestine, and therefore are relatively safe oral drug. They can adsorb bile acids in the intestine, reduce the reabsorption of bile acids, accelerate the excretion of bile acids, and reduce cholestasis, thereby showing a therapeutic effect on cholestatic liver diseases.

**3. Bile acid diarrhea**

Bile acid diarrhea (BAD), also known as "bile acid malabsorption (BAM)", is a chronic diarrhea caused by increased permeability of intestinal mucosa, increased intestinal juice secretion and increased intestinal peristalsis, which are due to the stimulation of excessive bile acids in the large intestine on the intestinal mucosa, wherein the excessive bile acids result from abnormal bile acid metabolism. In 1967, Hofmann from Mayo Clinic first proposed the concept of "biliary diarrhea and biliary enteropathy". Later, the bile acid-related diarrhea was divided into three types according to their causes: Type I is bile acid malabsorption caused by ileal diseases such as Crohn’s disease and ileal resection; Type II is idiopathic or primary BAD, the cause of which is unknown, and it is noted that 25%-50% of irritable bowel syndrome with predominant diarrhea (IBS-D) manifests BAM to certain extent; Type III is bile acid malabsorption caused by other gastrointestinal diseases, including cholecystectomy, overproliferation of bacteria in the small intestine, microscopic colitis, etc.

Depending on the severity of malabsorption, a large amount of bile acids can pass through the ileum into the colon, inducing several pathogenic effects. Bile acids, especially hydrophobic bile acids, are cytotoxic due to their detergent properties. Physiological concentration of bile acids in the colon has been shown to stimulate the propagation of movement waves, accelerate the transportation speed of colon and enhance the desire to defecate. Excessive bile acids also induce colonocytes to secrete chloride ions, leading to secretory diarrhea, which is the main feature of BAD.

The current recommended treatments of BAD include bile acid sequestrants, FXR agonists, loperamide, and low-fat diet. The first-generation bile acid sequestrant cholestyramine is a routine drug for clinical treatment of BAD and has a response rate of about 70% in several clinical trials. However, its taste is not good, resulting in poor medication compliance. The new generation bile acid sequestrant colesevelam hydrochloride, which was launched in 2000, has been used clinically as a first-line treatment and second-line treatment of BAD. However, additional bile acid sequestrants are still needed to provide patients with more treatment options.

The present invention aims to address the above-mentioned unmet market needs.

**Summary of the Invention**

In the first aspect, the present invention provides an ammonium salt polymer obtained from polymerization of a monomer of Formula (I) and a monomer of Formula (II),

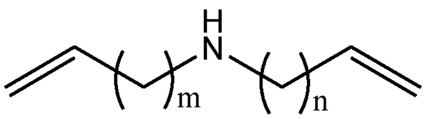

(I)

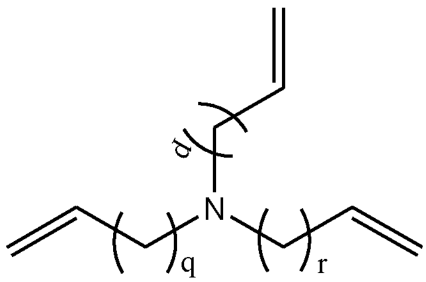

(II)

wherein:

m, n, p, q, r are each independently selected from 1, 2 or 3.

The ammonium salt polymer contains a monovalent, divalent or trivalent anion as counterion. The anion may be derived from organic or inorganic acids. The anion derived from inorganic acids include, but is not limited to: a monovalent anion, preferably selected from chloride ion (Cl), bicarbonate ion ($HCO_3^-$), bisulfate ion ($HSO_4^-$), acetate ion ($C_2H_3O_2^-$); a divalent anion, preferably selected from carbonate ion ($CO_3^{2-}$) , sulfate ion ($SO_4^2$); a trivalent anion, preferably phosphate ion ($PO_4^{3-}$); the anion derived from organic acids may be derived from malic acid, citric acid, fumaric acid, maleic acid, etc.

It will be understood by those skilled in the art that the ammonium cation and the anion contained in the polymer act as counterion to each other.

In an embodiment of the first aspect, the ammonium salt polymer is obtained from polymerization of the monomer of Formula (I) and the monomer of Formula (II) in a molar ratio of 10:0.5 to 10:15, preferably 10:1 to 10:12, more preferably 10:1 to 10:10, further preferably 10:1 to 10:4, most preferably 10:2.

In an embodiment of the first aspect, in the monomer of Formula (I), m and n are each independently selected from 1 and 2; preferably, m and n are both 1.

In an embodiment of the first aspect, in the monomer of Formula (II), p, q and r are each independently selected from 1 and 2; preferably, p, q and r are all 1.

The glass transition temperature of the ammonium salt polymer of the present invention is about 60°C to about 130°C, preferably about 70°C to 120°C, more preferably about 70°C to about 105°C. Preferably, the infrared (IR) spectrum of the ammonium salt polymer of the present invention has the following absorption bands ($cm^{-1}$): 2918, 2855, 2359, 2072, 1627, 1529, 1402, 1362, 1200, 1000, 909, 810. More preferably, the IR spectrum of the ammonium salt polymer of the present invention is shown in Figure 1.

Preferably, the $^{13}C$ solid-state Nuclear Magnetic Resonance (NMR) spectrum of the ammonium salt polymer of the present invention has the following lines (ppm): 28.2, 42.9, 50.5, 163.3. More preferably, the $^{13}C$ solid-state solid-state NMR spectrum of the ammonium salt polymer of the present invention is shown in Figure 2.

The ammonium salt polymer of the present invention has *in vitro* and *in vivo* bile acid absorption capability. For example, the *in vitro* bile acid absorption capability of the ammonium salt polymer of the present invention is 7-10 mmol/g, more preferably 7-8 mmol/g.

In the second aspect, the present invention provides a method for preparing the ammonium salt polymer of the present invention, comprising the following steps: (1) acidifying the monomer of Formula (I) and the monomer of Formula (II) using an acid; (2) polymerizing the acidified monomers obtained from step 1 to give the ammonium salt polymer of the present invention, as shown in the General Scheme 1.

General Scheme 1

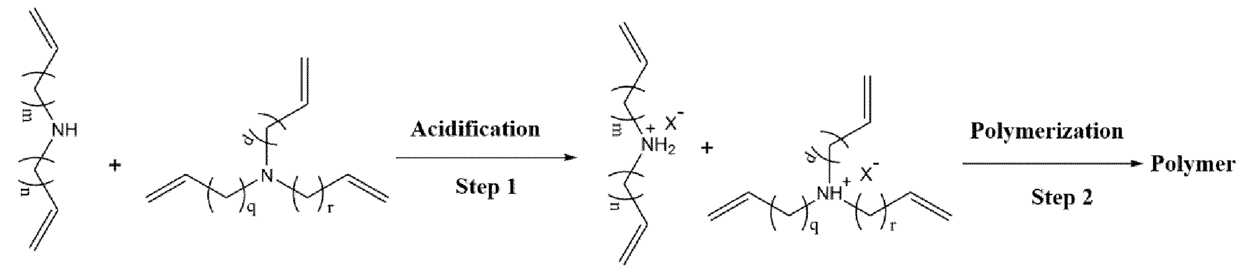


(I) (II)

wherein $X^-$ represents the aforesaid monovalent, divalent or trivalent anion as counterion of ammonium.

The acidification in step 1 may be performed by adding dropwise the monomer of Formula (I) and the monomer of Formula (II) in a molar ratio of 10:0.5 to 10:15, preferably 10:1 to 10:12, more preferably 10:1 to 10:10, further preferably 10:1 to 10:4, most preferably 10:2 to a suitable acid at a temperature of 30°C or lower, preferably, at a temperature of 15°C or lower, and after the addition, stirring for a period of time at a temperature of 5-30°C, preferably at a temperature of 10-20°C, to give a mixture of two acidified monomers.

The acids suitable for use in step 1 may be inorganic acid or organic acid, for example, hydrochloric acid, sulfuric acid, acetic acid, malic acid, citric acid, fumaric acid, maleic acid, etc.

The polymerization in step 2 may be performed by:

2.1. dissolving an initiator in water, adding the resulted solution into the mixture of acidified monomers obtained from step 1, to give an aqueous phase mixture;

2.2. in a separate container, dissolving an emulsifier in an organic solvent to give an oil phase mixture, wherein said organic solvent is immiscible with water and has a boiling point higher than the polymerization temperature, and said organic solvent is preferably selected from toluene, n-heptane, cyclohexane, xylene, n-octane and any mixture thereof;

2.3. adding the aqueous phase mixture into the oil phase mixture to perform the polymerization, to produce the desired ammonium salt polymer; preferably, the polymerization is performed, for example, at 40-100°C, preferably 50-85°C;

2.4. subjecting the reaction obtained from step 2.3 to a conventional work-up to isolate the ammonium salt polymer of the present invention. For example, the work-up may be performed by filtering the reaction, drying, and pulverizing to give the ammonium salt polymer of the present invention; preferably, the work-up may be performed by filtering the reaction, slurrying the wet filter cake once with methanol or ethanol and filtering, further slurrying the wet filter cake with purified water and filtering, repeating the slurrying with purified water and filtering process 2 to 3 times, drying, and pulverizing to give the ammonium salt polymer of the present invention.

optionally, step 2 may further comprise:

2.5. converting the wet filter cake obtained from step 2.4 or the ammonium salt polymer obtained from step 2.4 into an

ammonium salt polymer of the present invention containing another anion.

Step 2.5 may be performed by a conventional method known in the art. For example, when the acid used in step 1 is hydrochloric acid, the anion $X^-$ in the ammonium salt polymer obtained from step 2.3 is chloride anion. The polymer may be converted into an ammonium salt polymer containing another anion through step 2.5. For example, it may be converted into bicarbonate and/or carbonate by adding the wet filter cake obtained from step 2.4 or the ammonium salt polymer obtained from step 2.4 into a saturated aqueous sodium bicarbonate solution or an aqueous sodium carbonate solution, and after stirring for a period of time, filtering, drying and pulverizing to give an ammonium salt polymer, wherein the anion $X^-$ in the ammonium salt polymer is bicarbonate and/or carbonate anion.

There is no particular limitation on the initiator used in step 2.1, and the initiator may be any suitable initiator. For example, the initiator may be selected from 2,2'-azodiisobutyramidine dihydrochloride (V-50), 2,2'-azobis[2-(2-imidazolin-2-yl)propane]dihydrochloride (VA-044), ammonium persulfate, potassium persulfate, an aqueous hydrogen peroxide solution, and any mixture thereof.

There is no particular limitation on the emulsifier used in step 2.2, and the emulsifier may be any substance with emulsifying properties that is beneficial to mixing of the aqueous phase and the oil phase, such as a surfactant. For example, the emulsifier may be selected from polyoxyethylene sorbitan fatty acid esters (e.g., Tween 40, Tween 80, etc.), sorbitan fatty acid esters (e.g., Span 40, Span 60, Span 80 etc.).

In the third aspect, the present invention provides a pharmaceutical composition comprising the ammonium salt polymer of the present invention, and optionally a pharmaceutically acceptable carrier.

The pharmaceutical composition may be used as a bile acid sequestrant, especially for the treatment or prevention of a disease associated with excessive bile acids.

The pharmaceutical composition may be formulated into a solid formulation (including but not limited to capsules, tablets, pills, granules, powders) or a liquid formulation (including but not limited to suspensions) for oral administration by a method well known in the art.

For the oral formulation, the pharmaceutically acceptable carrier may be:

a) a filler, for example, lactose, dextrose, sucrose, mannitol, sorbitol, and cellulose;
b) a lubricant, for example, silica, talc, stearic acid, magnesium stearate or calcium stearate and/or polyethylene glycol;
c) a binder, for example, magnesium aluminum silicate, starch paste, gelatin, tragacanth, methylcellulose, sodium carboxymethylcellulose and/or polyvinylpyrrolidone;
d) a disintegrant, for example, starches, agar, alginic acid or its sodium salt, or effervescent mixtures; and/or
e) a coloring agent and/or a flavoring agent;
f) a suspending agent.

In the fourth aspect, the present invention provides use of the ammonium salt polymer of the present invention in the manufacture of a medicament as a bile acid sequestrant, as well as use of the ammonium salt polymer of the present invention in manufacture of a medicament for the treatment or prevention of a disease associated with excessive bile acids.

In the fifth aspect, the present invention provides a method for the treatment or prevention of a disease associated with excessive bile acids, comprising administering to a subject in need thereof an effective amount of the ammonium salt polymer of the present invention.

The disease associated with excessive bile acids described herein is selected from dyslipidemia (for example, hyperlipidemia), cholestatic liver diseases (for example, progressive familial intrahepatic cholestasis (PFIC), benign recurrent intrahepatic cholestasis (BRIC), inborn errors of bile acid synthesis, Alagille syndrome (ALGS), neonatal cholestasis caused by Cirtin deficiency, ARC syndrome, primary biliary cholangitis (PBC), primary sclerosing cholangitis (PSC), autoimmune hepatitis (AIH), drug-induced cholestasis (DRIC), biliary atresia (BA) and intrahepatic cholestasis of pregnancy (ICP)), bile acid diarrhea, and diabetes.

The inventors surprisingly found that the ammonium salt polymer of the present invention exhibited excellent bile acid adsorption capability, which is significantly better than that of colesevelam hydrochloride. Moreover, the *in vivo* effect is well consistent with the *in vitro* effect.

The ammonium salt polymer of the present invention will not be absorbed into the blood, and it adsorbs bile acids in the intestines and is excreted from the body with feces, thereby effectively reducing the re-absorption of bile acids in the intestine, improving cholestasis inside and outside the liver, preventing the progress of cholestatic liver diseases such as PSC and improving complications such as pruritus. Due to the non-systemic exposure property of the ammonium salt polymer of the present invention, the toxic side effects caused by small molecules such as Odevixibat entering into the blood are avoided. As a result, the ammonium salt polymer of the present invention exhibits good safety, can be administered for a long time, and has good compliance.

In addition, the preparation method of the ammonium salt polymer of the present invention is simple.

Specific embodiments

Embodiment 1. An ammonium salt polymer obtained from polymerization of a monomer of Formula (I) and a monomer of Formula (II),

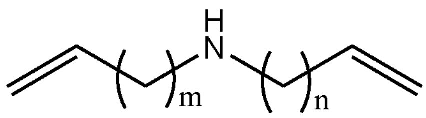

(I)

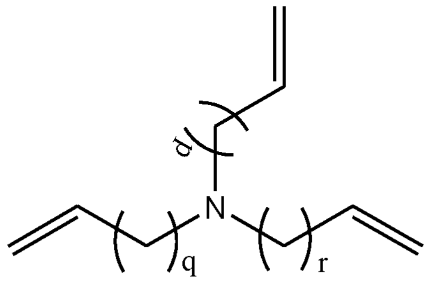

(II)

wherein:

m, n, p, q, r are each independently selected from 1, 2 or 3, and
the ammonium salt polymer comprises a monovalent, divalent or trivalent anion as counterion, and said anion is selected from chloride ion ($Cl^-$), bicarbonate ion ($HCO_3^-$), bisulfate ion ($HSO_4^-$), acetate ion ($C_2H_3O_2^-$), carbonate ion ($CO_3^{2-}$) , sulfate ion ($SO_4^{2-}$), phosphate ion ($PO_4^{3-}$), and anions derived from malic acid, citric acid, fumaric acid, maleic acid.

Embodiment 2. The polymer according to Embodiment 1, wherein m, n, p, q and r are each independently selected from 1 and 2, preferably wherein m, n, p, q and r are all 1.

Embodiment 3. The polymer according to Embodiment 1 or 2, wherein said ammonium salt polymer is obtained from polymerization of the monomer of Formula (I) and the monomer of Formula (II) in a molar ratio of 10:0.5 to 10:15, preferably 10:1 to 10:12, more preferably 10:1 to 10:10, further preferably 10:1 to 10:4, most preferably 10:2.

Embodiment 4. The polymer according to any one of Embodiments 1 to 3, wherein the glass transition temperature of the polymer is about 60°C to about 130°C, preferably about 70°C to 120°C, more preferably about 70°C to about 105°C.

Embodiment 5. The polymer according to any one of Embodiments 1 to 4, wherein the anion contained in the polymer is bicarbonate ion ($HCO_3^-$) and/or carbonate ion ($CO_3^{2-}$), and wherein m, n, p, q and r are all 1.

Embodiment 6. The polymer according to any one of Embodiments 1 to 5, wherein the polymer has an in vitro bile acid absorption capability of 7-10 mmol/g, preferably 7-8 mmol/g.

Embodiment 7. A method for preparing the polymer as defined in any one of Embodiments 1 to 6, comprising the steps of:

(1) acidifying the monomer of Formula (I) and the monomer of Formula (II) using an acid, preferably, the molar ratio of the monomer of Formula (I) to the monomer of Formula (II) is 10:0.5 to 10:15, preferably 10:1 to 10:12, more preferably 10:1 to 10:10, further preferably 10:1 to 10:4, most preferably 10:2;
(2) polymerizing the acidified monomers obtained from step (1), to give the desired ammonium salt polymer.

Embodiment 8. The method according to Embodiment 7, wherein step (1) is performed by adding dropwise the monomer of Formula (I) and the monomer of Formula (II) in a molar ratio of 10:0.5 to 10:15, preferably 10:1 to 10:12, more preferably 10:1 to 10:10, further preferably 10:1 to 10:4, most preferably 10:2 to a suitable acid at a temperature of 30°C or lower, preferably at a temperature of 15°C or lower, and after the completion of the addition, stirring for a period of time at a temperature of 5-30°C, preferably at a temperature of 10-20°C, to give a mixture of two acidified monomers. Embodiment 9. The method according to Embodiment 7 or 8, wherein the acid used in step (1) is selected from hydrochloric acid, sulfuric acid, acetic acid, malic acid, citric acid, maleic acid and fumaric acid.

Embodiment 10. The method according to any one of Embodiments 7 to 9, wherein step (2) comprises the sub-steps of:

(2.1) dissolving an initiator in water, adding the resulted solution into the mixture of acidified monomers obtained from

step (1), to give an aqueous phase mixture;

(2.2) in a separate container, dissolving an emulsifier in an organic solvent to give an oil phase mixture, wherein said organic solvent is immiscible with water and has a boiling point higher than the polymerization temperature, and said organic solvent is preferably selected from toluene, n-heptane, cyclohexane, xylene, n-octane and any mixture thereof;

(2.3) adding the aqueous phase mixture into the oil phase mixture to perform the polymerization, to produce the desired ammonium salt polymer;

(2.4) subjecting the reaction obtained from step (2.3) to a conventional work-up to isolate the ammonium salt polymer of the present invention; for example, the conventional work-up may be performed by filtering the reaction, drying, and pulverizing to give the ammonium salt polymer of the present invention; preferably, the conventional work-up may be performed by filtering the reaction, slurrying the wet filter cake once with methanol or ethanol and filtering, further slurrying the wet filter cake with purified water and filtering, repeating the slurrying with purified water and filtering process for 2 to 3 times, drying, and pulverizing to give the desired ammonium salt polymer; optionally

(2.5) converting the wet filter cake obtained from step (2.4) or the ammonium salt polymer obtained from step (2.4) into an ammonium salt polymer containing another anion.

Embodiment 11. A method for preparing the ammonium salt polymer as defined in Embodiment 5, comprising the steps of:

(1) acidifying the monomer of Formula (I) and the monomer of Formula (II) in a molar ratio of 10:0.5 to 10:15, preferably 10:1 to 10:12, more preferably 10:1 to 10:10, further preferably 10:1 to 10:4, most preferably 10:2 using concentrated hydrochloric acid, to give a mixture of two acidified monomers;

(2.1) dissolving an initiator in water, adding the resulted solution into the mixture of acidified monomers obtained from step (1), to give an aqueous phase mixture;

(2.2) in a separate container, dissolving an emulsifier in an organic solvent to give an oil phase mixture, wherein said organic solvent is immiscible with water and has a boiling point higher than the polymerization temperature, and said organic solvent is selected from toluene, n-heptane, cyclohexane, xylene, n-octane and any mixture thereof;

(2.3) adding the aqueous phase mixture into the oil phase mixture to perform the polymerization at a temperature of 40-100°C, preferably 50-85°C, to produce the desired ammonium salt polymer;

(2.4) filtering the reaction obtained from step (2.3), slurrying the wet filter cake once with methanol or ethanol and filtering, further slurrying the wet filter cake with purified water and filtering, repeating the slurrying with purified water and filtering process 2 to 3 times, to give a wet filter cake;

(2.5) adding the wet filter cake obtained from step (2.4) to a saturated aqueous sodium bicarbonate solution or an aqueous sodium carbonate solution, after stirring for a period of time, filtering, drying and pulverizing to give an ammonium salt polymer containing bicarbonate anion and/or carbonate anion.

Embodiment 12. The method according to any one of Embodiments 7 to 11, wherein the initiator is selected from 2,2'-azodiisobutyramidine dihydrochloride (V-50), 2,2'-azobis[2-(2-imidazolin-2-yl)propane]dihydrochloride (VA-044), ammonium persulfate, potassium persulfate, an aqueous hydrogen peroxide solution, and any mixture thereof; the emulsifier is selected from polyoxyethylene sorbitan fatty acid esters (e.g., Tween 40, Tween 80, etc.), sorbitan fatty acid esters (e.g., Span 40, Span 60, Span 80, etc.) and any mixture thereof.

Embodiment 13. The ammonium salt polymer according to any one of Embodiments 1 to 6, for use as a medicament, preferably for use as a medicament for the treatment or prevention of a disease associated with excessive bile acids, preferably the disease associated with excessive bile acids is selected from dyslipidemia (for example, hyperlipidemia), cholestatic liver diseases (for example, progressive familial intrahepatic cholestasis (PFIC), benign recurrent intrahepatic cholestasis (BRIC), inborn errors of bile acid synthesis, Alagille syndrome (ALGS), neonatal cholestasis caused by Cirtin deficiency, ARC syndrome, primary biliary cholangitis (PBC), primary sclerosing cholangitis (PSC), autoimmune hepatitis (AIH), drug-induced cholestasis (DRIC), biliary atresia (BA) and intrahepatic cholestasis of pregnancy (ICP)), bile acid diarrhea and diabetes.

Embodiment 14. A pharmaceutical composition comprising the polymer as defined in any one of Embodiments 1 to 6, and optionally a pharmaceutically acceptable carrier.

Embodiment 15. Use of the polymer according to any one of Embodiments 1 to 6 in the manufacture of a medicament as a bile acid sequestrant or a medicament for the treatment or prevention of a disease associated with excessive bile acids, which is preferably selected from dyslipidemia (for example, hyperlipidemia), cholestatic liver diseases (for example, progressive familial intrahepatic cholestasis (PFIC), benign recurrent intrahepatic cholestasis (BRIC), inborn errors of bile acid synthesis, Alagille syndrome (ALGS), neonatal cholestasis caused by Cirtin deficiency, ARC syndrome, primary biliary cholangitis (PBC), primary sclerosing cholangitis (PSC), autoimmune hepatitis (AIH), drug-induced cholestasis (DRIC), biliary atresia (BA) and intrahepatic cholestasis of pregnancy (ICP)), bile acid diarrhea

and diabetes.

Embodiment 16. A method for treating or preventing a disease associated with excessive bile acids, comprising administering to a subject in need thereof an effective amount of the polymer as defined in any one of Embodiments 1 to 6, wherein the disease is preferably selected from dyslipidemia (for example, hyperlipidemia), cholestatic liver diseases (for example, progressive familial intrahepatic cholestasis (PFIC), benign recurrent intrahepatic cholestasis (BRIC), inborn errors of bile acid synthesis, Alagille syndrome (ALGS), neonatal cholestasis caused by Cirtin deficiency, ARC syndrome, primary biliary cholangitis (PBC), primary sclerosing cholangitis (PSC), autoimmune hepatitis (AIH), drug-induced cholestasis (DRIC), biliary atresia (BA) and intrahepatic cholestasis of pregnancy (ICP)), bile acid diarrhea and diabetes.

Embodiment 17. The invention as hereinbefore described.

## Definitions

As used herein, the term "subject" refers to animals. Preferably, the subject is mammals, such as primates (for example, humans, monkeys, chimpanzees), cattle, sheep, goats, horses, dogs, cats, rabbits, rats, mice, etc. More preferably, the subject is humans.

As used herein, the term "treatment" or "treating" refers to alleviation or cure of a disease or its symptoms, or prevention of the progression of a disease or its symptoms.

As used herein, the term "prevention" or "preventing" refers to delay or prevention of the development of a disease or its symptoms.

As used herein, the term "effective amount" refers to an amount of the ammonium salt polymer of the present invention that can significantly reduce bile acids in the body and prevent the occurrence of a disease associated with excessive bile acids or its symptoms or improve a disease associated with excessive bile acids or its symptoms. The "effective amount" of the ammonium salt polymer of the present invention depends on a variety of factors, including but not limited to the type and severity of the disease to be treated or prevented, the general health condition and response of patients, etc. Generally, the "effective amount" of the ammonium salt polymer of the present invention is 0.5-24 g/day for a person of 70kg body weight. However, if desired, the ammonium salt polymer of the present invention may be administered in an amount outside the aforesaid dosage range. The effective amount for a particular patient can be readily determined by a physician.

As used herein, the term "about" represents that the numerical value given thereafter can be extended up or down by 20%, preferably by 10%. For example, "about 100" means 80 to 120, preferably 90 to 110; "about 60°C" means 48°C to 72°C, preferably 54°C to 66°C.

## Examples

The following examples illustrate the invention and should not be construed as limiting the scope of the present invention in any way. The disclosed data (e.g., amounts, temperatures, etc.) are provided as accurate as possible, but some experimental errors and deviations may exist. Unless otherwise stated, temperatures are given in degree Celsius, and pressures are at or near atmospheric pressure. Unless otherwise stated, all reagents used in the Examples are commercially available.

The abbreviations used herein have the following meanings:

| | |
|---|---|
| DAA | diallylamine |
| TAA | triallylamine |
| DAA-Cl | diallyl ammonium chloride |
| TAA-Cl | triallyl ammonium chloride |
| DMDAAC | diallyl dimethyl ammonium chloride |
| BTHB | 1-bromo-6-(trimethylammonium) hexyl bromide |
| VA-044 | 2,2'-azobis[2-(2-imidazolin-2-yl)propane] dihydrochloride |
| V-50 | 2,2'-azodiisobutyramidine dihydrochloride |
| Span 60 | Span-60, sorbitan monostearate |
| RPM | revolutions per minute |
| NaOH | sodium hydroxide |
| $NaHCO_3$ | sodium bicarbonate |
| DDC | 3,5-diethoxycarbonyl-1,4-dihydrocollidine |
| TBA | total bile acids |
| TBIL | total bilirubin |
| Concentrated hydrochloric acid | hydrochloric acid with a concentration of 36%-38% |
| ANIT | $\alpha$-naphthylisothiocyanate |

| | |
|---|---|
| GC | gas chromatography |
| CMC-Na | carboxymethyl cellulose sodium |
| SDS | sodium dodecyl sulfate |

## Example 1

(Preparation of the polymer WS012-A according to the general scheme 1)

1.76 kg Concentrated hydrochloric acid was added to a reaction flask, and 1.46 kg (15mol) DAA and 0.41kg (3mol) TAA were added to the reaction flask dropwise, keeping the internal temperature not more than 15°C. After completion of the addition, the reaction was stirred at 10-20°C for about 0.5-1 hour. 0.146 kg V-50 and 1.46 kg water were added to a beaker. After stirring to dissolve, the solution in the reaction flask was added to the beaker, and the mixture was stirred to mix uniformly. 8.76 kg n-heptane and 0.133 kg Span 60 were added to a reactor, and heated to 40-50°C under stirring to dissolve with the rotation speed of mechanical stirring set at 200-250 RPM. The mass in the beaker was added to the reactor, heated to 50-55°C, and stirred at 50-55°C for 15-24 hours. After the GC detection of the reaction solution showed that the conversion of the monomers DAA and TAA was almost complete, the reaction mixture was filtered. The wet filter cake was slurried with about 5.84 kg ethanol for 1-2 h followed by filtering, then the wet filter cake was slurried with about 18.0-20.0 kg water for 1-2 h followed by filtering, and the slurrying with the purified water and filtering process was repeated 2 to 3 times. The wet filter cake was added to about 30.0 kg saturated aqueous $NaHCO_3$ solution and stirred at 25-35°C for 18-24 hours followed by filtering, and the slurrying and filtering process was repeated 2 times. The wet filter cake was slurried with about 18.0-20.0 kg water for 1-2 hours followed by filtering, and the slurrying and filtering process was repeated 2 to 3 times. Then, the wet filter cake was rinsed with purified water. The filer cake was air-dried at 45-55°C, then pulverized and sieved to give 1.8 kg of the polymer WS012-A as a light yellow solid.

Attempts to dissolve the prepared polymer with a variety of conventional solvents such as water, methanol, N,N-dimethylformamide, dimethyl sulfoxide showed that the polymer was not soluble in all of these solvents.

The prepared polymer WS012-A was characterized using SHIMADZU IRSpirit-T fourier transform infrared spectrophotometer according to the infrared spectrophotometry ("Chinese Pharmacopoeia" 2020 Edition, Volume IV, General Chapters, 0402). The obtained IR spectrum is shown in Figure 1, which has the following characteristic absorption bands ($cm^{-1}$): 2918, 2855, 2359, 2072, 1627, 1529, 1402, 1362, 1200, 1000, 909, 810.

The IR spectrum showed the presence of the structures such as methylene, tertiary ammonium salt, secondary ammonium salt, C-N, carbonate in the product.

The prepared polymer WS012-A in the present Example was detected using Jnm-600ECZ solid-state NMR spectrometer. The test sample was filled into 3.2mm ZrO2 rotor with the experimental parameters: a magic angle spinning speed of 15 KHz, a recycle delay time of 5 seconds, 1024 scans at 293K, and the chemical shift of the carbonyl group of glycine at 176.04 ppm as an external standard. The obtained $^{13}C$ solid-state NMR spectrum is shown in Figure 2, and has the following lines (ppm): 28.2, 42.9, 50.5, 163.3.

The $^{13}C$ solid-state NMR spectrum showed the presence of tertiary carbon, methylene carbon, C-N and carbonate in the test sample.

The prepared product WS012-A was detected by differential scanning calorimetry (DSC). Instrument: METTLER TOLEDO DSC3 differential scanning calorimeter. Analytical Methods: $N_2$ atmosphere, 50ml/min. Scanning procedure: heating from 30°C to 180°C at 10°C/min and keeping the temperature for 5min, then cooling to 30°C at 10°C/min and keeping the temperature for 5min, heating to 180°C at 30°C/min and recording the second heating curve. The characteristic glass transition temperature was determined from the curve. An aluminum sample tray was used. The obtained DSC curve is shown in Figure 3. The results showed that the glass transition temperature of the polymer WS012-A is 99.5°C.

## Example 2

(An alternative method for preparing the polymer WS012-A according to the general scheme 1) 101.54 g concentrated hydrochloric acid was added to the reaction flask 1. 82.59g (0.85 mol) DAA and 23.33g (0.17 mol) TAA were added to the reaction flask 1 dropwise, keeping the internal temperature not more than 15°C. After completion of the addition, the reaction was stirred at 10-20°C for about 0.5-1 hour. 5.93g VA-044 and 54.0g water were added to a conical flask, and stirred to dissolve. Then, the aqueous solution was added to the reaction flask 1 and stirred to mix uniformly.

580 mL toluene and 4.1g Span 60 were added to the reaction flask 2, and heated to 40-50°C under stirring to dissolve with the rotation speed of mechanical stirring set at 300-400RPM. The mass in the reaction flask 1 was added to the reaction flask 2, heated to 50-55°C, and stirred at 50-55°C for 15-24 hours. After the GC detection of the reaction solution showed that the conversion of DAA and TAA was almost complete, the internal temperature of the reaction was increased to 80-85°C, and the reaction was stirred at 80-85°C for 1-2 hours, and then cooled and filtered. The wet filter cake

was slurried with about 1L water for 0.5-1 h following by filtering, and the slurrying and filtering process was repeated 3 to 5 times. The wet filter cake was added to 500 mL 10% of aqueous NaOH solution, and stirred at ambient temperature for about 8-12 h. Then, the reaction mixture was filtered. The wet filter cake was slurried with about 1L water for 0.5-1 h following by filtering. The wet filter cake was added to about 1L saturated aqueous $NaHCO_3$ solution, and stirred at 20-30°C for 12-18 hours following by filtering, and these procedures were repeated 1 to 2 times. The wet filter cake was slurried with about 1L water for 0.5-1 h following by filtering, and the slurrying and filtering process was repeated 2 to 3 times. The wet filter cake was rinsed with purified water. The filter cake was air-dried at 45-55°C, pulverized, and sieved to give 80g of the polymer WS012-A as a light yellow solid.

The product was detected using SHIMADZU IRSpirit-T fourier transform infrared spectrophotometer and Jnm-600ECZ solid-state NMR spectrometer in Example 1. The result showed that the IR spectrum and $^{13}C$ solid-state NMR spectrum of the polymer were the same as those of the polymer in Example 1 (spectra were not given).

In addition, the solubility properties of the product of the present Example in various solvents were also determined. The results showed that, like the product in Example 1, the polymer was not soluble in any solvent.

## Example 3

(An alternative method for preparing the polymer WS012-A according to the general scheme 1) 101.54 g concentrated hydrochloric acid was added to the reaction flask 1. 82.59g (0.85 mol) DAA and 23.33g (0.17 mol) TAA were added to the reaction flask 1 dropwise, keeping the internal temperature not more than 15°C. After completion of the addition, the reaction was stirred at 10-20°C for about 0.5-1 h. 5.93g VA-044 and 54.0g water were added to a conical flask, and stirred to dissolve. Then, the aqueous solution was added to the reaction flask 1, and stirred to mix uniformly. 600mL n-heptane and 8.36g Span 60 were added to the reaction flask 2, and heated to 40-50°C under stirring to dissolve with the rotation speed of mechanical stirring set at 300-400RPM. The mass in the reaction flask 1 was added to the reaction flask 2, heated to 50-55°C, and stirred at 50-55°C for 15-24 hours. After the GC detection of the reaction solution showed that the conversion of DAA and TAA was almost complete, the reaction mixture was filtered. The wet filter cake was slurried with about 1L methanol for 0.5-1 h followed by filtering, then the wet filter cake was further slurried with about 1L water for 0.5-1 h followed by filtering, and the slurrying and filtering process was repeated 2 to 3 times. The wet filter cake was added to 500mL 10% aqueous NaOH solution, and stirred at ambient temperature for about 8-12 hours. Then, the reaction mixture was filtered. The wet filter cake was slurried with about 1L water for 0.5-1 h followed by filtering. The wet filter cake was added to about 1L saturated aqueous $NaHCO_3$ solution and stirred at 20-30°C for 12-18 hours followed by filtering, and these procedures were repeated 1 to 2 times. The wet filter cake was slurried with about 1L water for 0.5-1 h followed by filtering, and the slurrying and filtering process was repeated 2 to 3 times. Then, the wet product was rinsed with purified water. The filter cake was air-dried at 45-55°C, pulverized and sieved to give 75g of the polymer WS012-A as a light yellow solid.

The product was detected using SHIMADZU IRSpirit-T fourier transform infrared spectrophotometer and Jnm-600ECZ solid-state NMR spectrometer of Example 1. The results showed that the IR spectrum and $^{13}C$ solid-state NMR spectrum of the polymer were the same as those of the polymer in Example 1 (spectra were not given).

In addition, the solubility properties of the product of the present Example in various solvents were also determined. The results showed that, like the product in Example 1, the polymer was not soluble in any solvent.

## Example 4

(An alternative method for preparing the polymer WS012-A according to the general scheme 1) 101.54 g concentrated hydrochloric acid was added to the reaction flask 1. 82.59g (0.85 mol) DAA and 23.33g (0.17 mol) TAA were added to the reaction flask 1 dropwise, keeping the internal temperature not more than 15°C. After completion of the addition, the reaction was stirred at 10-20°C for about 0.5-1 h. 5.93g VA-044 and 54.0g water were added to a conical flask, and stirred to dissolve. Then, the aqueous solution was added to the reaction flask 1, and stirred to mix uniformly. 480mL cyclohexane and 7.17g Span 60 were added to the reaction flask 2, and heated to 40-50°C under stirring to dissolve with the rotation speed of mechanical stirring set at 300-400 RPM. The mass in the reaction flask 1 was added to the reaction flask 2, heated to 50-55°C, and stirred at 50-55°C for 15-24 hours. After the GC detection of the reaction solution showed that the conversion of DAA and TAA was almost complete, the reaction mixture was filtered. The wet filter cake was slurried with about 1L methanol for 0.5-1 h followed by filtering, then the wet filter cake was further slurried with about 1L water for 0.5-1 h followed by filtering, and the slurrying and filtering process was repeated 2 to 3 times. The wet filter cake was added to 500mL 10% aqueous NaOH solution, and stirred at ambient temperature for about 8-12 h. Then, the reaction mixture was filtered. The wet filter cake was slurried with about 1L water for 0.5-1 h followed by filtering. The wet filter cake was added to about 1L saturated aqueous $NaHCO_3$ solution and stirred at 20-30°C for 12 to 18 h followed by filtering, and these procedures were repeated for 1 to 2 times. The wet filter cake was slurried with about 1L water for 0.5-1 h followed by filtering, and the slurrying and filtering process was repeated 2 to 3 times. Then, the wet product was rinsed with purified

water. The filter cake was air-dried at 45-55°C, pulverized and sieved to give 90g of the polymer WS012-A as a light yellow solid.

The product was detected using SHIMADZU IRSpirit-T fourier transform infrared spectrophotometer and Jnm-600ECZ solid-state NMR spectrometer of Example 1. The results showed that the IR spectrum and $^{13}C$ solid-state NMR spectrum of the polymer were the same as those of the polymer in Example 1 (spectra were not given).

In addition, the solubility properties of the product of the present Example in various solvents were also determined. The results showed that, like the product in Example 1, the polymer was not soluble in any solvent.

**Example 5**

(Preparation of the polymer WS012-B)

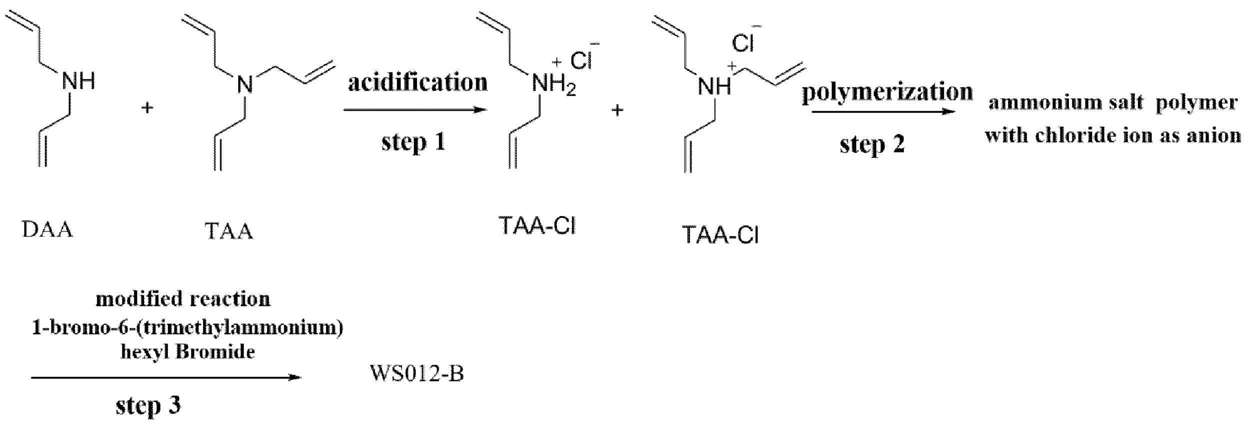


DAA TAA TAA-Cl TAA-Cl

304.6 g concentrated hydrochloric acid was added to the reaction flask 1. 247.7g (2.55mol) DAA and 69.99g (0.51mol) TAA were added to the reaction flask 1 dropwise, keeping the internal temperature not more than 15°C. After completion of the addition, the reaction was stirred at 10-20°C for about 0.5-1 h. 17.79g VA-044 and 180g water were added to a conical flask, and stirred to dissolve. Then, the aqueous solution was added to the reaction flask 1, and stirred to mix uniformly.

1740 mL toluene and 12.3g Span 60 were added to the reaction flask 2, and heated to 40-50°C under stirring to dissolve with the rotation speed of mechanical stirring set at 300-400RPM. The mass in reaction flask 1 was added to the reaction flask 2, heated to 50-55°C, and stirred at 50-55°C for 15-24 hours. After the GC detection of the reaction solution showed that the reaction is complete, the internal temperature in the reaction flask was increased to 80-85°C, and the reaction was stirred at 80-85°C for 1-2 hours, cooled and filtered. The wet filter cake was slurried with about 4L water for 0.5-1 h followed by filtering, and the slurrying and filtering process was repeated 3-5 times. The wet filter cake was added to 4L 10% aqueous NaOH solution, and stirred at ambient temperature for about 8-12 hours. Then, the reaction mixture was filtered. The wet filter cake was slurried with about 1L water for 0.5-1 h followed by filtering. The filter cake was air-dried at 45-55°C, pulverized and sieved to give a solid, which was the ammonium salt polymer with chloride ion as anion.

The said solid, 55.0g 1-bromo-6-(trimethylammonium)hexyl bromide (0.18mol) and 2.4L methanol were added to a reaction flask, heated to 65-70°C, and stirred at 65-70°C for 24-36 hours. After the reaction is complete, the reaction mixture was filtered. The wet filter cake was slurried with about 1L methanol for 0.5-1 h followed by filtering, then the wet filter cake was further slurried with about 1L water for 0.5-1 h followed by filtering, and the slurrying with purified water and filtering process was repeated 2 to 3 times. The wet filter cake was added to about 4L saturated aqueous $NaHCO_3$ solution, stirred at 20-30°C for 12-18 hours, and then filtered, and these procedures were repeated for 1 to 2 times. The wet filter cake was slurried with about 1L water for 0.5-1 h followed by filtering, and the slurrying and filtering process was repeated 2 to 3 times. Then, the wet product was rinsed with purified water. The filter cake was air-dried at 45-55°C. The dried product was pulverized and sieved to give 170g of the polymer WS012-B as a light yellow solid.

The product of the present Example was detected using SHIMADZU IRSpirit-T fourier transform infrared spectrophotometer of Example 1. The IR spectrum is shown in Figure 4.

In addition, the solubility properties of the product of the present Example in various solvents were also determined. The results showed that, like the product in Example 1, the polymer was not soluble in any solvent.

The product WS012-B prepared in the present Example was determined using the differential scanning calorimetry (DSC) of Example 1. The obtained DSC curve is shown in Figure 5. The results showed that the glass

transition temperature of the polymer WS012-B was 58.8°C.

**Example 6**

(Preparation of the ammonium salt polymer WS012-C)

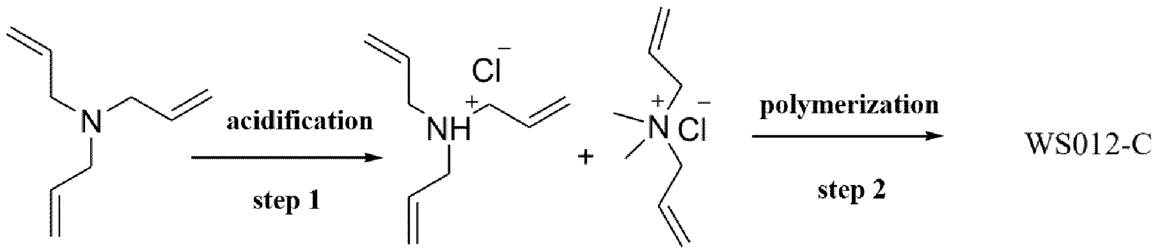


TAA TAA-Cl DMDAAC

178.9 g concentrated hydrochloric acid was added to a 3L reaction flask. 246.8g (1.8 mol) TAA was added to the reaction flask dropwise, keeping the internal temperature not more than 15°C. After completion of the addition, the reaction was stirred at 10-20°C for about 0.5-1 h. 15.7g VA-044 and 45g water were added to a conical flask, and stirred to dissolve. Then, the aqueous solution was added to the reaction flask 1, further added with 242.5g (0.9 mol) 60% aqueous dimethyldiallylammonium chloride solution, and stirred to mix uniformly.

1.6L toluene and 11.8g Span 60 were added to the reaction flask, heated to 40-50oC under stirring to dissolve with the rotation speed of mechanical stirring set at 200-300RPM. The mass in the reaction flask was added to a reactor. The temperature was increased to 50-55oC, and the reaction was stirred at 50-55oC for 15-24 hours. After the GC detection of the reaction solution showed that the conversion of TAA was almost complete, the reaction mixture was filtered. The wet filter cake was slurried with about 3L methanol for 0.5-1 h followed by filtering, then the wet filter cake was further slurried with about 10L water for 0.5-1 h followed by filtering, and the slurrying and filtering process was repeated 1 to 2 times. The wet filter cake was added to about 7.5L saturated aqueous $NaHCO_3$ solution and stirred at 20-30°C for 12-18 hours followed by filtering, and these procedures were repeated for 1 to 2 times. The wet filter cake was slurried with about 10L water for 0.5-1 h followed by filtering, and the slurring and filtering process was repeated 2 to 3 times. Then, the wet product was rinsed with purified water. The filter cake was air-dried at 45-55°C, pulverized and sieved to give about 200g of the polymer WS012-C as a light yellow solid.

The product of the present Example was detected using SHIMADZU IRSpirit-T fourier transform infrared spectrophotometer of Example 1. The IR spectrum is shown in Figure 6.

The product WS012-C prepared in the present Example was determined using the differential scanning calorimetry (DSC) of Example 1. The obtained DSC curve is shown in Figure 7. The result showed that the glass transition temperature of the polymer WS012-C was 177.8°C.

**Examples 7 to 13**

In Example 7-13, polymers were prepared according to the method described in Example 1 using different amounts of DAA and/or TAA respectively, and the products were detected using the detection methods described in Example 1. The amounts of each reactant and experimental results in Example 7-13 are shown as follows:

| Example # | Amount of DAA and/or TAA | Amount of concentrated hydrochloric acid | Amount of V50 | Amount of n-heptane | Amount of Span 60 | Glass transition temperature |
|---|---|---|---|---|---|---|
| 7 | DAA= 68.0g(0.7mol) | 69.7g | 5.7g | 343g | 5.1g | - |
| 8 | DAA:TAA= 68.0g(0.7mol):9.6g(0.07mol) | 76.6g | 6.3g | 370g | 5.6g | 69.9°C |

(continued)

| Example # | Amount of DAA and/or TAA | Amount of concentrated hydrochloric acid | Amount of V50 | Amount of n-heptane | Amount of Span 60 | Glass transition temperature |
|---|---|---|---|---|---|---|
| 9 | DAA:TAA= 48.6g(0.5mol):27.4g(0.2mol) | 69.7g | 5.7g | 346g | 5.2g | 88.4°C |
| 10 | DAA:TAA= 48.6g(0.5mol):47.2g(0.3mol) | 79.6g | 6.5g | 400g | 6.0g | 101.0°C |
| 11 | DAA:TAA= 38.8g(0.4mol):43.9g(0.32mol) | 71.7g | 5.8g | 364g | 5.5g | 99.2°C |
| 12 | DAA:TAA= 33.7g(0.35mol):48.0g(0.35mol) | 69.7g | 5.7g | 356g | 5.3g | 92.9°C |
| 13 | TAA= 96.0g(0.7mol) | 69.7g | 5.7g | 376g | 5.6g | 155.4°C |

The above results showed that no solid polymer product was formed when DAA was used as the only monomer for the reaction in Example 7, and polymers were formed in Examples 8-13 as light yellow solid. The products were detected using the DSC method described in Example 1, and the obtained glass transition temperatures are shown in the last column of the above table.

**Example 14**

(In vitro bile acid adsorption experiment)

1. Preparation of solutions:

Mixed bile acids solution: 6 mmol sodium glycocholate, 6 mmol sodium glycochenodeoxycholate, and 2 mmol sodium taurodeoxycholate were contained in each 1L of 0.68% phosphate buffered saline to give the mixed bile acids solution (pH 6.8).

Linear solutions: the mixed bile acids solution was diluted to obtain linear solutions 1 to 5, which contain respectively 0.06, 0.18, 0.3, 0.48 or 0.6 mmol sodium glycocholate, 0.06, 0.18, 0.3, 0.48 or 0.6 mmol sodium glycochenodeoxycholate, and 0.02, 0.06, 0.1, 0.16, or 0.2 mmol sodium taurodeoxycholate in each 1L of purified water.

Test solution: 100 mg the test sample was weighed accurately into a 250 ml iodine flask, added with 100 ml of the mixed bile acids solution and stirred with a magnetic stirring bar in a water bath (37°C ± 0.5°C). 5 ml of the solution was sampled at 5 h and left at room temperature. (The solution was left at room temperature for at least 5 minutes after sampling. Then, the iodine flask was tilted and shaked to make the water droplets on the upper part of the iodine flask fall down. The iodine flask was shaked and rotated for at least 15 seconds.) The sample was filtered. 1.0 ml of the filtrate was taken into a 10 ml volumetric flask, diluted to the graduation marking with purified water, shaked uniformly for injection.

Chromatography conditions

| | |
|---|---|
| **Instrument** | Agilent 1260 or equivalent |
| **Column** | Waters X-briage (150×4.6mm, 5μm) or another column with equivalent performance |
| **Flow rate** | 1.0 ml/min |
| **Column temperature** | 25°C |
| **Detection wavelength** | 210 nm |
| **Injection volume** | 10 μl |

(continued)

| | |
|---|---|
| **Mobile phase** | A phosphate buffer (about 3.4g of potassium monobasic phosphate was taken and weighed accurately, 1000 ml of water was added to dissolve. The pH value was adjusted to 6.50$\pm$0.05): Acetonitrile = 65:35. Isocratic elution. |
| **Run time** | 10 min |

2. Determination of the bile acid binding capacity using the standard curve method.

The bile acid concentrations in the linear solutions (unit: mmol/L) as the X-coordinate and the peak areas as the Y-coordinate were used to fit the linear regression equation Y=aX+b.

$$\text{bile acid binding capacity (mmol/g)} = \frac{\left(C_0 - \frac{A_{SPL} - b}{a} \times S\right) \times V}{m \times (1 - W)}$$

wherein:

a is the slope of the linear regression equation;
b is the intercept of the linear regression equation;
$A_{SPL}$ is the peak area of the test solution;
$C_0$ is the initial concentration of each bile acid in the test solution, μmol/L;
V is the volume of the added bile acid binding solution, 0.1L;
S is the dilution volume of the test sample, 10ml;
m is the weighed amount of the test sample;
W is the moisture value of the test sample, %.

3. Experimental results

| Test samples | Reactants and molar ratio thereof | Total bile acid adsorption capacity in vitro |
|---|---|---|
| Colesevelam hydrochloride | commercially purchased | 4.820mmol/g |
| Example 1 (WS012-A) | DAA:TAA=10:2 | 7.704mmol/g |
| Example 2 (WS012-A) | DAA:TAA=10:2 | 7.573mmol/g |
| Example 3 (WS012-A) | DAA:TAA=10:2 | 7.719mmol/g |
| Example 4 (WS012-A) | DAA:TAA=10:2 | 7.758mmol/g |
| Example 5 (WS012-B) | DAA:TAA:BTBH =10:2:0.7 | 6.413mmol/g |
| Example 6 (WS012-C) | TAA:DMDAAC=10:5 | 5.086mmol/g |
| Example 8 (WS012-A) | DAA:TAA=10:1 | 7.332mmol/g |
| Example 9 (WS012-A) | DAA:TAA=10:4 | 7.352mmol/g |
| Example 10 (WS012-A) | DAA:TAA=10:6 | 7.069mmol/g |
| Example 11 (WS012-A) | DAA:TAA=10:8 | 7.089mmol/g |
| Example 12 (WS012-A) | DAA:TAA=10:10 | 7.122mmol/g |
| Example 13 (WS012-D) | TAA | 4.309mmol/g |

The experimental results above showed that the ammonium salt polymer WS012-A of the present invention has the highest total bile acid adsorption capacity in vitro, and is superior to the ammonium salt polymer WS012-B, the ammonium salt polymer WS012-C, the ammonium salt polymer WS012-D, and colesevelam hydrochloride.

**Example 15**

(Effects of WS012-A on DDC-induced primary sclerosing cholangitis (PSC) in mice)

1. Experimental procedures:

After an acclimation period, 36 male C57BL/6J mice (18-22g, 6-8 weeks old) were randomly grouped into 6 experimental groups according to their body weight, namely normal control group, model control group, positive drug control group 1, positive drug control group 2, low dose WS012-A group and high dose WS012-A group, with 6 animals in each group. Vehicle (0.5% aqueous CMC-Na solution) was administered to the normal control group and the model control group, 0.3 mg/kg Odevixibat (BioChemPartner) was administered to the positive drug control group 1, 300 mg/kg colesevelam hydrochloride (Formosa laboratories, Inc.) was administered to the positive drug control group 2, and 300 mg/kg and 1000 mg/kg of WS012-A prepared in Example 2 were respectively administered to the low-dose WS012-A group and the high-dose WS012-A group.

Modeling was performed according to a known method (Peter Fickert, et.al, The American Journal of Pathology, Vol. 171, No. 2, August 2007, pages 525-536). Specifically, except for the mice in the normal control group, mice in the other groups were fed with 0.1% DDC for 14 consecutive days with ad libitum consumption of food and water. Administration once a day was conducted while modeling for consecutive 14 days. The specified test samples or the vehicle were administered in a volume of 20 ml/kg by gavage in all groups. On the day after the last administration, blood was collected from the animals under anesthesia for the detections of alkaline phosphatase (ALP), total bile acids (TBA), and total bilirubin (TBIL). Feces were collected during 24 hours on Days 13-14, and TBA content in the feces was detected. The feces were pretreated as follows: 50 mg of feces from each case was weighted, added with 2% aqueous SDS solution in the ration 1:20 w/v, ultrasound for 20 minutes, and centrifuged at 1000 rpm for 5 minutes. The supernatant was taken for detection.

All parameters were represented as mean ± SEM. The parameters of animals before and after administration in various groups were plotted using Graph Pad Prism 8 software. Data were analyzed using SPSS 22.0 statistical software. The parameters were subjected to Test for Homogeneity of Variance using Levene's test method. When the variances were homogeneous ($P \geq 0.05$), the difference between groups was compared using Dunnett's & LSD method in one-way analysis of variance (ANOVA); when the variances were heterogeneous ($P<0.05$), the difference between groups was compared using Mann-Whitney U Test (M-W method) in Kruskal-Wallis H rank sum test (K-W method).

2. Experimental results:

Effects of test samples on blood indexes of 0.1% DDC diet-induced PSC mice model (Mean±SEM, n=6)

| Group | Dose (mg/kg) | ALP (U/L) | TBA (μmol/L) | TBIL (μmol/L) |
|---|---|---|---|---|
| Normal group | / | 147±8*** | 4.68±1.62*** | 5.0±0.1** |
| Model group | / | 1534±143 | 215.05±22.25 | 52.4±9.2 |
| Odevixibat | 0.3 | 686±64*** | 89.25±37.42* | 19.4±0.9* |
| Colesevelam hydrochloride | 300 | 1670±100 | 202.96±32.72 | 73.1±6.3 |
| low dose WS012-A | 300 | 1485±131 | 170.03±35.60 | 38.8±7.8## |
| high dose WS012-A | 1000 | 1172±100*## | 110.65±8.44*# | 27.0±3.4*### |

Compared with the model group, *P<0.05, **P<0.01, ***P<0.001; compared with the colesevelam hydrochloride group, #P<0.05, ##P<0.01, ###P<0.001

The experimental results of the present Example are shown in Figure 8.

Figure 8A showed that compared with the normal group, the serum ALP in the model group was significantly elevated (P<0.001); compared with the model group, the serum ALP in the Odevixibat treatment group was significantly decreased (P<0.001), the serum ALP in the colesevelam hydrochloride treatment group was slightly elevated without statistical significance, and the serum ALP in both the low-dose WS012-A treatment group and the high-dose WS012-A treatment group was markedly decreased, exhibiting a dose-dependent relationship to some extent, in which the decrease of ALP in the high-dose WS012-A treatment group was statistically significant (P<0.05).

Figure 8B showed that compared with the normal group, the serum TBA in the model group was significantly elevated (P<0.001); compared with the model group, the serum TBA in the Odevixibat treatment group was significantly decreased (P<0.05), the serum TBA in the colesevelam hydrochloride treatment group was not substantially decreased,

and the serum TBA in both the low-dose WS012-A treatment group and the high-dose group was markedly decreased, exhibiting a dose-dependent relationship to some extent, in which the decrease of TBA in the high-dose WS012-A treatment group was statistically significant (P<0.05).

Figure 8C showed that compared with the normal group, the serum TBIL in the model group was significantly elevated (P<0.001); compared with the model group, the serum TBIL in the Odevixibat treatment group was significantly decreased (P<0.05), the serum TBIL in the colesevelam hydrochloride treatment group was elevated, the serum TBIL in both the low-dose WS012-A treatment group and the high-dose WS012-A treatment group were markedly decreased, exhibiting a dose-dependent relationship to some extent, in which the decrease of TBIL in the high-dose WS012-A treatment group was statistically significant (P<0.05); and the serum TBILs in the low-dose WS012-A treatment group and the high-dose WS012-A treatment group were significantly lower than that of the colesevelam hydrochloride treatment group (P<0.01 and P<0.001, respectively).

Figure 8D showed that compared with the normal group, the TBA excretion in the feces in the model group was significantly decreased (P<0.001); compared with the model group, the TBA excretion in the feces in the Odevixibat treatment group was significantly elevated (P<0.001), the TBA excretion in the feces in the colesevelam hydrochloride treatment group was significantly elevated (P<0.05), and the TBA excretion in both the feces in the low-dose WS012-A treatment group and the high-dose WS012-A treatment group was significantly elevated (P<0.05).

The above results showed:

① Compared with the normal group, the serum ALP, TBA and TBIL in the model group were all significantly elevated, and the TBA excretion in feces was significantly decreased, indicating that the DDC feeding led to significant changes of the physiological indexes of cholestasis in mice and the model was successfully established.

② Compared with the model group, in the Odevixibat (a first-line drug for the treatment of progressive familial intrahepatic cholestasis (PFIC)) treatment group, the serum ALP, TBA and TBIL were significantly decreased, and the TBA excretion in feces was significantly elevated, indicating that this drug has a good therapeutic effect on cholestasis.

③ Compared with the model group, in the colesevelam hydrochloride treatment group, the serum ALP and TBIL were elevated, which represented unfavorable effects on the treatment of cholestasis; the serum TBA was not substantially decreased, showing that colesevelam hydrochloride had no significant influence on this parameter; and the TBA excretion in feces was significantly elevated, which is beneficial to the treatment of cholestasis. Overall, colesevelam hydrochloride did not exhibit marked improvement effect on cholestasis and is not suitable for the treatment of cholestasis.

④ Compared with the model group, the serum ALP, TBA, and TBIL in both the low-dose WS012-A treatment group and the high-dose WS012-A treatment group were markedly decreased, exhibiting a dose-dependent relationship to some extent, in which the decreases of ALP, TBA, and TBIL in the high-dose WS012-A treatment group were statistically significant; the elevation of TBA excretion in feces in both the low-dose WS012-A treatment group and the high-dose WS012-A treatment group was statistically significant. Overall, WS012-A exhibited therapeutic effects on cholestasis, and the therapeutic effects are dose-dependent.

⑤ By comparing the colesevelam hydrochloride treatment group and the low-dose WS012-A treatment group, it can be seen that the effects on these four investigated parameters produced by the low dose of WS012-A were better than those produced by colesevelam hydrochloride.

3. Experimental conclusions:

In the DDC feeding-induced PSC mice model, by oral administration of WS012-A by gavage at 300 mg/kg and 1000 mg/kg once a day for 14 consecutive days, WS012-A increased the TBA excretion in feces and decreased the serum ALP, TBA and TBIL, and relieved the pathological changes of liver cholestasis, and the effects were better than that of colesevelam hydrochloride, which also belongs to polymeric bile acid sequestrants.

**Example 16**

(Effects of WS012-A on ANIT-induced acute cholestasis rat model)

1. Experimental procedures:

After an acclimation period, 48 male SD rats (180-220g, 6-8 weeks old) were randomly grouped into 6 experimental groups according to their body weight, namely the normal control group, the model control group, the positive drug control group 1, the positive drug control group 2, the WS012-A group, the WS012-C group, with 8 animals in each group. Vehicle (0.5% aqueous CMC-Na solution) was administered to the normal control group and the model control

group, 0.3 mg/kg Odevixibat was administered to the positive drug control group 1, 1000 mg/kg colesevelam hydrochloride was administered to the positive drug control group 2, 1000 mg/kg of WS012-A prepared in Example 1 was administered to the WS012-A group, and 1000 mg/kg of WS012-C prepared in Example 6 was administered to the WS012-C group. The specified test samples or the vehicle were administered once a day in a volume of 20 ml/kg by gavage for 7 days in all groups. Modeling was performed according to a method known in the art (Yoshiji Ohta, et al, Toxicology 139 (1999) 265-275). Specifically, except for the rats in the normal control group, rats in the other groups were injected intraperitoneally with a single dose of ANIT at 80mg/kg on Day 4 of the administration. On Day 7 of the administration (72±1h after the injection of the modeling agent ANIT), the animals were anesthetized, and whole blood was collected from the intrajugular venous plexus and centrifuged to collect serum for the detection of alanine aminotransferase (ALT), aspartate aminotransferase (AST), alkaline phosphatase (ALP), and total bile acids (TBA).

All parameters were represented as mean ± SEM. The parameters of animals before and after administration in various groups were plotted using Graph Pad Prism 8 software. Data were analyzed using SPSS 22.0 statistical software. The parameters were subjected to Test for Homogeneity of Variance using Levene's test method. When the variances were homogeneous (P≥0.05), the difference between groups was compared using Dunnett's & LSD method in one-way analysis of variance (ANOVA); when the variances were heterogeneous (P<0.05), the difference between groups was compared using Mann-Whitney U Test (M-W method) in Kruskal-Wallis H rank sum test (K-W method).

2. Experimental results:

Blood indexes after the completion of the administration (Mean±SEM, n=8)

| Group | Dose mg/kg | **AST** U/L | **ALT** U/L | **ALP** U/L | **TBA** μmol/L |
|---|---|---|---|---|---|
| **Normal group** | / | 128±9*** | 53±4*** | 357±22*** | 34.28±4.22** |
| **Model group** | / | 363±39 | 361±45 | 853±34 | 109.04±16.96 |
| **Odevixibat** | 0.3 | 317±43 | 235±25 | 674±26** | 33.57±1.21** |
| **Colesevelam hydrochloride** | 1000 | 289±50 | 211±31* | 662±32** | 57.19±7.76* |
| **WS012-A** | 1000 | 239±33* | 162±30** | 485±72** | 43.79±13.26* |
| **WS012-C** | 1000 | 247±15* | 163±24** | 569±37** | 40.44±7.94** # |
| Compared with the model group, *P<0.05, **P<0.01, ***P<0.001; compared with the colesevelam hydrochloride treatment group, #P<0.05 | | | | | |

The experimental results of the present Example are shown in Figure 9.

Figure 9A showed that compared with the normal group, the serum ALT in the model group was significantly elevated (P<0.001); compared with the model group, the serum ALT in the Odevixibat treatment group, the colesevelam hydrochloride treatment group, the WS012-A treatment group, and the WS012-C treatment group was decreased, in which the decrease in the colesevelam hydrochloride treatment group (P<0.05), the WS012-A treatment group (P<0.01), and the WS012-C treatment group (P<0.01) were statistically significant. As for as the decrease of serum ALT, WS012-A and WS012-C exhibited better effect than colesevelam hydrochloride.

Figure 9B showed that compared with the normal group, the serum AST in the model group was significantly elevated (P<0.001); compared with the model group, the serum AST in the Odevixibat treatment group, the colesevelam hydrochloride treatment group, the WS012-A treatment group, and the WS012-C treatment group was decreased, in which the decrease in the colesevelam hydrochloride treatment group was not statistically significant, while the decrease in both the WS012-A treatment group and WS012-C treatment group was statistically significant (P<0.05), and the WS012-A group exhibited the best effect.

Figure 9C showed that compared with the normal group, the serum ALP in the model group was significantly elevated (P<0.001); compared with the model group, the serum ALP in the Odevixibat treatment group, the colesevelam hydrochloride treatment group, the WS012-A treatment group, and the WS012-C treatment group was significantly decreased (P<0.01), in which the WS012-A group exhibited the best effect.

Figure 9D showed that compared with the normal group, the serum TBA in the model group was significantly elevated (P<0.01); compared with the model group, the serum TBA in the Odevixibat treatment group, the colesevelam hydrochloride treatment group, the WS012-A treatment group, and the WS012-C treatment group was significantly decreased (P<0.05 or P<0.01), and the serum TBA in the WS012-A treatment group and the WS012-C treatment group was lower than that of the colesevelam hydrochloride treatment group.

The above results showed:

① Compared with the normal group, the serum ALT, AST, ALP, TBA in the model group were significantly elevated (P<0.05-0.001), indicating that the ANIT feeding led to significant changes in the physiological indexes of cholestasis and the model was successfully established.

② Compared with the model group, the serum ALT, AST, ALP, TBA in the Odevixibat treatment group, the colesevelam hydrochloride treatment group and the WS012-A treatment group were decreased, in which the decreases of the serum ALP and TBA in the Odevixibat treatment group were statistically significant (P<0.01), the decreases of the serum ALT, ALP, TBA in the colesevelam hydrochloride treatment group were statistically significant (P<0.05-0.01), the decreases of the serum ALT, ALP, TBA in the WS012-A treatment group and the WS012-C treatment group were statistically significant (P<0.05-0.01).

3. Experimental conclusions:

In the acute cholestasis rat model induced by a single intraperitoneal injection of ANIT (80 mg/kg), by oral administration of WS012-A or WS012-C by gavage at 1000 mg/kg once a day for 7 consecutive days, WS012-A significantly decreased the serum ALT (P<0.01), the serum ALP (P<0.01), the serum TBA (P<0.05), and decreased the serum AST to some extent; WS012-C also significantly decreased the serum ALT (P<0.01), AST (P<0.05), ALP (P<0.01), TBA (P<0.01). Overall, both WS012-A and WS012-C exhibited the effect of improving cholestasis, and their effects were better than that of colesevelam hydrochloride, which also belongs to polymeric bile acid sequestrants, and also better than that of the small molecule drug Odevixibat (an IBAT inhibitor).

**Example 17**

(Effects of WS012-A on high fat diet-induced hyperlipidemia in golden hamster)

1. Experimental procedures:

After an acclimation period of about 7 days, 8 of 48 male golden hamsters (100-150g, 6-7 weeks old) were grouped into the blank control group and fed with basic diet continuously, and the remaining animals were grouped into the experimental groups and feed with high-fat diet (basic diet + 15% lard + 20% sucrose + 1.2% cholesterol + 0.2% sodium cholate). Conditions of animals such as vigor and diet were observed regularly. After feeding with high-fat diet for 7 weeks, the animals were fasted overnight. On the next day, blood was taken from the orbital venous plexus for the detection of TC, TG, and LDL-C contents in the plasma. The golden hamsters were grouped according to the detection results. Based on blood biochemical indexes and body weight, 32 golden hamsters with hyperlipidemia were screened and grouped into 4 groups on average, namely the model control group (Model), the test sample group 1 (WS012-A prepared in Example 2, 1000mg/kg), the test sample group 2 (WS012-B prepared in Example 5, 1000 mg/kg), the positive drug control group (colesevelam hydrochloride, 1000 mg/kg). The specified test samples or the vehicle were administered once a day in a volume of 20.0 ml/kg by gavage for 4 weeks. The blank control group was fed with normal diet, and the other groups were fed with high fat diet continuously. During the administration period, the hamsters were weighed once a week and the changes in body weight were observed. Blood was collected before administration and 1, 2, 3, and 4 weeks after administration for the detection of total cholesterol (TC), triglycerides (TG) and low-density lipoprotein cholesterol (LDL-C) in the serum; feces were collected 2 weeks and 4 weeks after administration (within 24 hours after administration) for the detection of the total bile acids (TBA) in the feces supernatant. After the last feces collection, the animals were euthanized and the livers were harvested for observation.

All parameters were represented as mean ± SEM. The parameters of animals before and after administration in various groups were plotted using Graph Pad Prism 8 software. Data were analyzed using SPSS 22.0 statistical software. The parameters were subjected to Test for Homogeneity of Variance using Levene's test method. When the variances were homogeneous (P≥0.05), the difference between groups was compared using Dunnett's & LSD method in one-way analysis of variance (ANOVA); when the variances were heterogeneous (P<0.05), the difference between groups was compared using Mann-Whitney U Test (M-W method) in Kruskal-Wallis H rank sum test (K-W method).

2. Experimental results:

The experimental results of the present Example are shown in Figure 10.

Figure 10A showed that compared with the normal group, the total cholesterol (TC) in the blood in the model group was significantly elevated before administration and after administration for 1 to 4 weeks (P<0.001), and the total cholesterol (TC) was elevated slowly as the induction time of high-fat diet is extended. Compared with the model group, the TC concentration in the positive control (colesevelam hydrochloride) group was decreased to some extent due to the drug administration, but the decrease of TC in the colesevelam hydrochloride treatment group was statistically significant only

after the administration for 3 week (P<0.01), and the decreases of TC at the other sampling time points were not significantly different from that of the model group; after administration for 1 to 4 weeks, the TC levels in the WS012-A treatment group were reduced significantly (P< 0.05-0.001); after administration for 2 to 4 weeks, the TC levels in the WS012-B treatment group were reduced significantly (P<0.05-0.001). Compared with the positive control (colesevelam hydrochloride) group, the TC levels in both WS012-A and WS012-B treatment groups were reduced significantly after administration for 4 weeks (P<0.01). The above results showed that both WS012-A and WS012-B exhibited a better effect than colesevelam hydrochloride in reducing TC, and WS012-A exhibited the best effect.

Figure 10B showed that compared with the normal group, triglycerides (TG) in the blood in the model group was elevated significantly before administration and after administration for 1 to 4 weeks (P<0.05). Compared with the model group, the TG in the colesevelam hydrochloride treatment group was not significantly different after drug treatment; and the TC in the WS012-A treatment group was lower than that in the model group during the entire administration period. Compared with the model group, the TG in the WS012-A treatment group was decreased significantly after administration for 1, 2 and 4 weeks (P<0.05); the TG in the WS012-B treatment group was not significantly different from that in the model group during the entire administration period (P>0.05). Compared with the positive control (colesevelam hydrochloride) group, the TG in the WS012-A treatment group was significantly lower after administration for 4 weeks (P<0.01). The above results showed that WS012-A exhibited an excellent effect in reducing TG, which was better than that of colesevelam hydrochloride.

Figure 10C showed that compared with the normal group, the low-density lipoprotein (LDL-C) in the blood in the model group was elevated significantly before administration and after administration for 1 to 4 weeks (P<0.001). Compared with the model group, the LDL-C concentration in the colesevelam hydrochloride treatment group was decreased to some extent after drug administration, but the decrease of LDL-C in the colesevelam hydrochloride treatment group was significant only after administration for 3 weeks (P<0.05), and the decreases of LDL-C at the other sampling time points were not significantly different from that of the model group (P>0.05); the LDL-C levels in the WS012-A treatment group were all reduced significantly after administration for 1 to 4 weeks (P<0.05-0.001); the LDL-C levels in the WS012-B treatment group were lower than those of the model group during the entire administration period, but the decrease of LDL-C in the WS012-B treatment group was significant only after administration for 1 week and for 4 weeks (P <0.01-0.001). Compared with the colesevelam hydrochloride treatment group, the LDL-C levels in the WS012-A treatment group were reduced significantly after administration for 1, 2 and 4 weeks (P<0.05-0.001); the LDL-C levels in the WS012-B treatment group were reduced significantly after administration for 1 and 4 weeks. The above results showed that both WS012-A and WS012-B exhibited a significant effect in decreasing LDL-C, which is better than that of colesevelam hydrochloride, and WS012-A exhibited the best effect.

Figure 10D and Figure 10E showed that compared with the normal group, the liver weight and liver index in the model group increased significantly after the completion of drug administration (P< 0.001), indicating that the model was successfully established. Compared with the model group, the liver weight and liver index in the WS012-A and WS012-B treatment groups were reduced after the completion of drug administration, in which the decrease of the liver weight and liver index in the WS012-A treatment group was statistically significant (P<0.05). Compared with the positive control colesevelam hydrochloride group, the liver weight and liver index in the WS012-A and WS012-B treatment groups were decreased after the completion of drug administration, but only the decreases of the liver weight (P<0.05) and liver index (P<0.01) in the WS012-A treatment group were statistically significant. The above results showed that both WS012-A and WS012-B exhibited an effect in improving hepatomegaly induced by the high-fat diet, but only the effect of WS012-A is significantly better than that of colesevelam hydrochloride.

Figure 10F showed that the livers of the animals in the normal control were bright red, and had a smooth surface and soft texture. The livers of the golden hamsters in the model control group had significantly increased size, tended to turn white, were swollen overall, and showed slightly rounded edges and brittle texture. The liver tissue of the animals in the positive control colesevelam hydrochloride group was slightly different from that of the model group, but the difference was small. The livers of the animals in the positive control group had significantly increased size, tended to turn white, were swollen overall, and showed slightly rounded edges and brittle texture, while no decrease was found in liver weight and liver index. Compared with the model control group and the normal control group, the livers of the animals in the WS012-A treatment group and the WS012-B treatment group were Turkey red, and tended to be white in the edges, and had an overall size between the size of the model control group and the size of the normal control group. Among the test samples, WS012- A exhibited the best effect.

3. Experimental conclusions:

In the golden hamster hyperlipidemia model induced by high-fat diet for 7 weeks, oral administration of WS012-A or WS012-B by gavage at 1000 mg/kg once a day for 4 constitutive weeks decreased the levels of blood lipids (TC, TG, LDL-C), alleviated the fat pathological changes of liver (liver weight, liver index, gross observation of liver). Among the test samples, WS012-A exhibited the best effect, which was better than that of colesevelam hydrochloride, which also belong

to polymeric bile acid sequestrants.

Based on the above experimental results of the present disclosure, it can be concluded that the ammonium salt polymer of the present invention exhibited an excellent adsorption effect for bile acids in vitro and in vivo, which is better than that of colesevelam hydrochloride and that of other ammonium salt polymers WS012-B and WS012-C prepared by the present disclosure.

## Brief Description of the Drawings

Figure 1 shows an IR spectrum of the ammonium salt polymer WS012-A prepared in Example 1.
Figure 2 shows a $^{13}C$ NMR spectrum of the ammonium salt polymer WS012-A prepared in Example 1.
Figure 3 shows a DSC curve of the ammonium salt polymer WS012-A prepared in Example 1.
Figure 4 shows an IR spectrum of the ammonium salt polymer WS012-B prepared in Example 5.
Figure 5 shows a DSC curve of the ammonium salt polymer WS012-B prepared in Example 5.
Figure 6 shows an IR spectrum of the ammonium salt polymer WS012-C prepared in Example 6.
Figure 7 shows a DSC pattern of the ammonium salt polymer WS012-C prepared in Example 6.
Figure 8 shows the results of DDC-induced primary sclerosing cholangitis mice model of Example 15, wherein, "Normal" represents the normal group, "Model" represented the model group, "Odevixibat" represents the Odevixibat treatment group, "Coleveselam" represents the colesevelam hydrochloride treatment group, "WS012-A-Low" represents the treatment group of the low dose of WS012-A prepared in Example 2, and "WS012-A-High" represents the treatment group of the high dose of WS012-A prepared in Example 2.
Figure 9 shows the results of ANIT-induced primary biliary cholangitis rat model of Example 16, wherein, "Normal" represents the normal group, "Model" represents the model group, "Odevixibat" represents the Odevixibat treatment group, Coleveselam represents the colesevelam hydrochloride treatment group, "WS012-A" represents the treatment group of WS012-A prepared in Example 1, and "WS012-C" represents the treatment group of WS012-C prepared in Example 6.
Figure 10 shows the results of high fat diet-induced hyperlipidemia golden hamster model of Example 17, wherein, "Normal" represents the normal group, "Model" represents the model group, "Coleveselam" represents the colesevelam hydrochloride treatment group, "WS012-A" represents the treatment group of WS012-A prepared in Example 2, and "WS012-B" represents the treatment group of WS012-B prepared in Example 5.

The specific embodiments and examples given herein are only for purpose of illustrating the invention and do not constitute any limitation on the scope defined by the claims. Based on the present disclosure, those skilled in the art can obviously understand equivalent variations of the technical solutions of the present disclosure. These variations are also encompassed by the present disclosure.

## Claims

1. An ammonium salt polymer obtained from polymerization of a monomer of Formula (I) and a monomer of Formula (II),

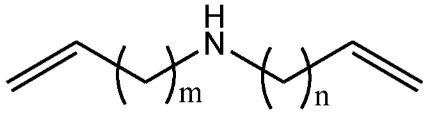
(I)

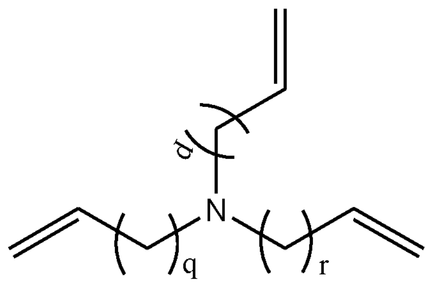
(II)

wherein:

m, n, p, q, r are each independently selected from 1, 2 or 3, and

the ammonium salt polymer contains a monovalent, divalent or trivalent anion as counterion, and said anion is selected from chloride ion ($Cl^-$), bicarbonate ion ($HCO_3^-$), bisulfate ion ($HSO_4^-$), acetate ion ($C_2H_3O_2^-$), carbonate ion ($CO_3^{2-}$) , sulfate ion ($SO_4^{2-}$), phosphate ion ($PO_4^{3-}$), and anions derived from malic acid, citric acid, fumaric acid, maleic acid.

**2.** The polymer according to claim 1, wherein m, n, p, q and r are each independently selected from 1 and 2, preferably wherein m, n, p, q and r are all 1.

**3.** The polymer according to claim 1 or 2, wherein said ammonium salt polymer is obtained from polymerization of the monomer of Formula (I) and the monomer of Formula (II) in a molar ratio of 10:0.5 to 10:15, preferably 10:1 to 10:12, more preferably 10:1 to 10:10, further preferably 10:1 to 10:4, most preferably 10:2.

**4.** The polymer according to any one of claims 1 to 3, wherein the glass transition temperature of the polymer is about 60°C to about 130°C, preferably about 70°C to 120°C, more preferably about 70°C to about 105°C.

**5.** The polymer according to any one of claims 1 to 4, wherein the anion contained in the polymer is bicarbonate ion ($HCO_3^-$) and/or carbonate ion ($CO_3^{2-}$), and wherein m, n, p, q and r are all 1.

**6.** The polymer according to any one of claims 1 to 5, wherein the polymer has an in vitro bile acid absorption capability of 7-10 mmol/g, preferably 7-8 mmol/g.

**7.** A method for preparing the polymer as defined in any one of claims 1 to 6, comprising the steps of:

(1) acidifying the monomer of Formula (I) and the monomer of Formula (II) using an acid, preferably, the molar ratio of the monomer of Formula (I) to the monomer of Formula (II) is 10:0.5 to 10:15, preferably 10:1 to 10:12, more preferably 10:1 to 10:10, further preferably 10:1 to 10:4, most preferably 10:2;
(2) polymerizing the acidified monomers obtained from step (1), to give the desired ammonium salt polymer.

**8.** The method according to claim 7, wherein step (1) is performed by adding dropwise the monomer of Formula (I) and the monomer of Formula (II) in a molar ratio of 10:0.5 to 10:15, preferably 10:1 to 10:12, more preferably 10:1 to 10:10, further preferably 10:1 to 10:4, most preferably 10:2 to a suitable acid at a temperature of 30°C or lower, preferably at a temperature of 15°C or lower, and after the completion of the addition, stirring for a period of time at a temperature of 5-30°C, preferably at a temperature of 10-20°C, to give a mixture of two acidified monomers.

**9.** The method according to claim 7 or 8, wherein the acid used in step (1) is selected from hydrochloric acid, sulfuric acid, acetic acid, malic acid, citric acid, maleic acid and fumaric acid.

**10.** The method according to any one of claims 7 to 9, wherein step (2) comprises the sub-steps of:

(2.1) dissolving an initiator in water, adding the resulted solution into the mixture of acidified monomers obtained from step (1), to give an aqueous phase mixture;
(2.2) in a separate container, dissolving an emulsifier in an organic solvent to give an oil phase mixture, wherein said organic solvent is immiscible with water and has a boiling point higher than the polymerization temperature, and said organic solvent is preferably selected from toluene, n-heptane, cyclohexane, xylene, n-octane and any mixture thereof;
(2.3) adding the aqueous phase mixture into the oil phase mixture to perform the polymerization, to produce the desired ammonium salt polymer;
(2.4) subjecting the reaction obtained from step (2.3) to a conventional work-up to isolate the desired ammonium salt polymer;
optionally
(2.5) converting the wet filter cake obtained from step (2.4) or the ammonium salt polymer obtained from step (2.4) into an ammonium salt polymer containing another anion.

**11.** A method for preparing the ammonium salt polymer as defined in claim 5, comprising the steps of:

(1) acidifying the monomer of Formula (I) and the monomer of Formula (II) in a molar ratio of 10:0.5 to 10:15, preferably 10:1 to 10:12, more preferably 10:1 to 10:10, further preferably 10:1 to 10:4, most preferably 10:2 using concentrated hydrochloric acid, to give a mixture of two acidified monomers;

(2.1) dissolving an initiator in water, adding the resulted solution into the mixture of acidified monomers obtained from step (1), to give an aqueous phase mixture;
(2.2) in a separate container, dissolving an emulsifier in an organic solvent to give an oil phase mixture, wherein said organic solvent is immiscible with water and has a boiling point higher than the polymerization temperature, and said organic solvent is selected from toluene, n-heptane, cyclohexane, xylene, n-octane and any mixture thereof;
(2.3) adding the aqueous phase mixture into the oil phase mixture to perform the polymerization at a temperature of 40-100°C, preferably 50-85°C, to produce the desired ammonium salt polymer;
(2.4) filtering the reaction obtained from step (2.3), slurrying the wet filter cake once with methanol or ethanol and filtering, further slurrying the wet filter cake with purified water and filtering, repeating the slurrying with purified water and filtering process 2 to 3 times, to give a wet filter cake;
(2.5) adding the wet filter cake obtained from step (2.4) to a saturated aqueous sodium bicarbonate solution or an aqueous sodium carbonate solution, after stirring for a period of time, filtering, drying and pulverizing to give an ammonium salt polymer containing bicarbonate anion and/or carbonate anion.

**12.** The method according to any one of claims 7 to 11, wherein the initiator is selected from 2,2'-azodiisobutyramidine dihydrochloride (V-50), 2,2'-azobis[2-(2-imidazolin-2-yl)propane]dihydrochloride (VA-044), ammonium persulfate, potassium persulfate, an aqueous hydrogen peroxide solution, and any mixture thereof; the emulsifier is selected from polyoxyethylene sorbitan fatty acid esters (e.g., Tween 40, Tween 80, etc.), sorbitan fatty acid esters (e.g., Span 40, Span 60, Span 80, etc.) and any mixture thereof.

**13.** The ammonium salt polymer according to any one of claims 1 to 6, for use as a medicament, preferably for use as a medicament for the treatment or prevention of a disease associated with excessive bile acids, preferably the disease associated with excessive bile acids is selected from dyslipidemia (for example, hyperlipidemia), cholestatic liver diseases (for example, progressive familial intrahepatic cholestasis (PFIC), benign recurrent intrahepatic cholestasis (BRIC), inborn errors of bile acid synthesis, Alagille syndrome (ALGS), neonatal cholestasis caused by Cirtin deficiency, ARC syndrome, primary biliary cholangitis (PBC), primary sclerosing cholangitis (PSC), autoimmune hepatitis (AIH), drug-induced cholestasis (DRIC), biliary atresia (BA) and intrahepatic cholestasis of pregnancy (ICP)), bile acid diarrhea and diabetes.

**14.** A pharmaceutical composition comprising the polymer as defined in any one of claims 1 to 6, and optionally a pharmaceutically acceptable carrier.

**15.** A method for treating or preventing a disease associated with excessive bile acids, comprising administering to a subject in need thereof an effective amount of the polymer as defined in any one of claims 1 to 6, wherein the disease is preferably selected from dyslipidemia (for example, hyperlipidemia), cholestatic liver diseases (for example, progressive familial intrahepatic cholestasis (PFIC), benign recurrent intrahepatic cholestasis (BRIC), inborn errors of bile acid synthesis, Alagille syndrome (ALGS), neonatal cholestasis caused by Cirtin deficiency, ARC syndrome, primary biliary cholangitis (PBC), primary sclerosing cholangitis (PSC), autoimmune hepatitis (AIH), drug-induced cholestasis (DRIC), biliary atresia (BA) and intrahepatic cholestasis of pregnancy (ICP)), bile acid diarrhea and diabetes.

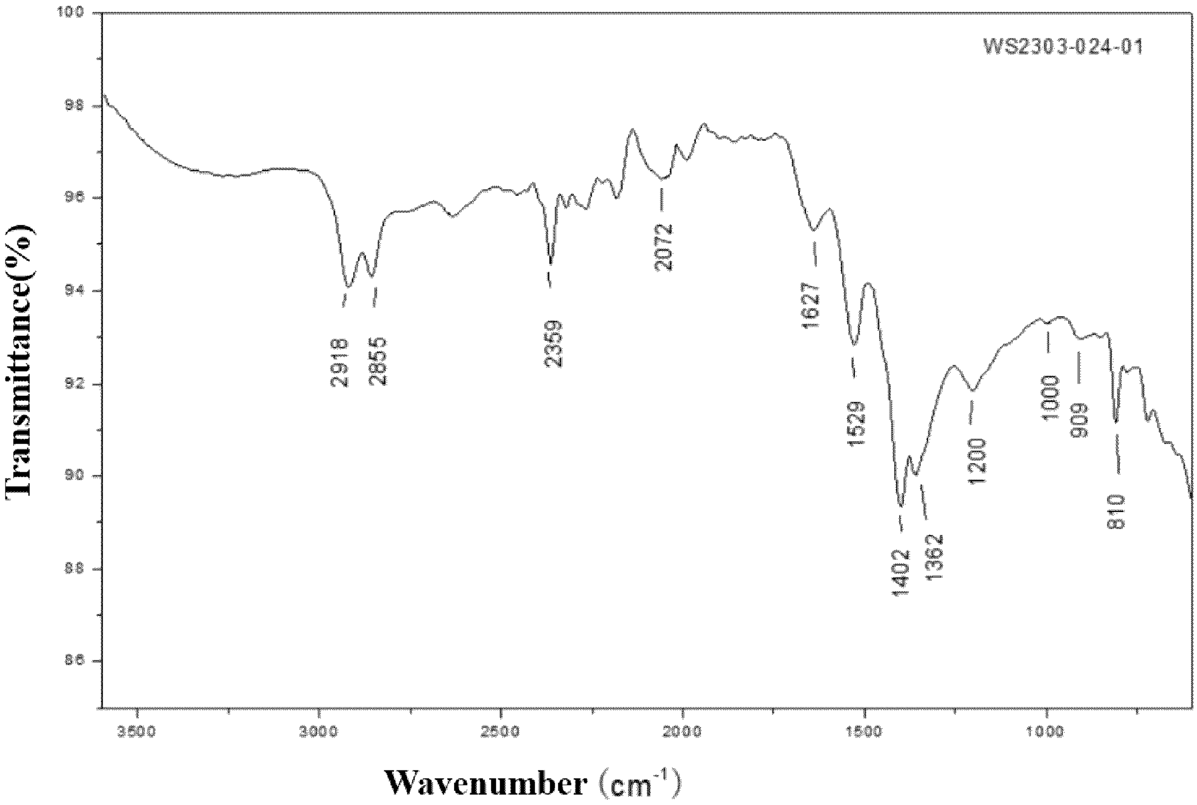


**Figure 1**

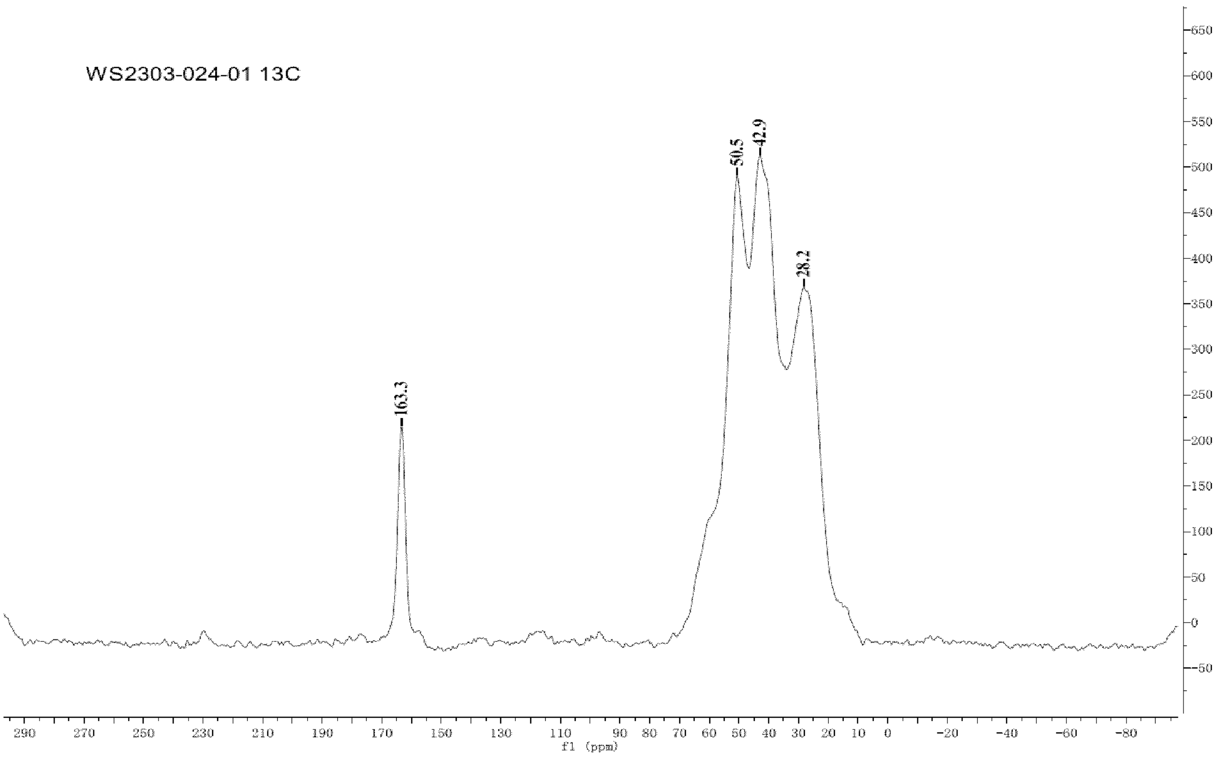


**Figure 2**

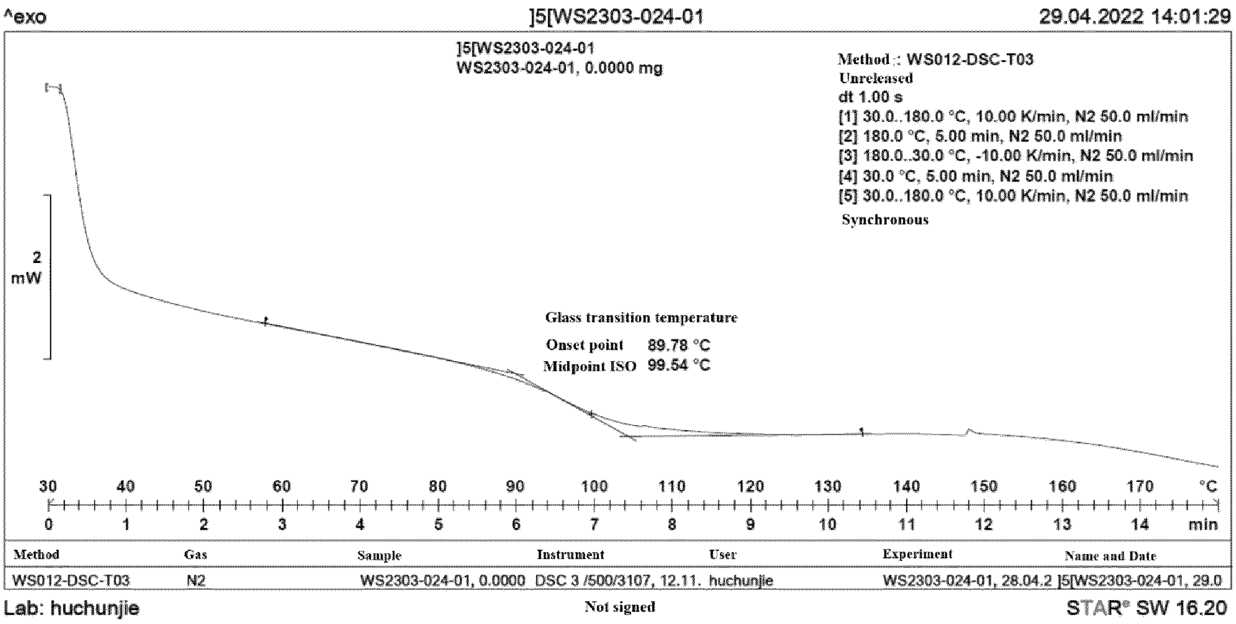
^exo
]5[WS2303-024-01
29.04.2022 14:01:29
]5[WS2303-024-01
WS2303-024-01, 0.0000 mg
Method : WS012-DSC-T03
Unreleased
dt 1.00 s
[1] 30.0..180.0 °C, 10.00 K/min, N2 50.0 ml/min
[2] 180.0 °C, 5.00 min, N2 50.0 ml/min
[3] 180.0..30.0 °C, -10.00 K/min, N2 50.0 ml/min
[4] 30.0 °C, 5.00 min, N2 50.0 ml/min
[5] 30.0..180.0 °C, 10.00 K/min, N2 50.0 ml/min
Synchronous
2 mW
Glass transition temperature
Onset point 89.78 °C
Midpoint ISO 99.54 °C
30 40 50 60 70 80 90 100 110 120 130 140 150 160 170 °C
0 1 2 3 4 5 6 7 8 9 10 11 12 13 14 min
Method Gas Sample Instrument User Experiment Name and Date
WS012-DSC-T03 N2 WS2303-024-01, 0.0000 DSC 3 /500/3107, 12.11. huchunjie WS2303-024-01, 28.04.2 ]5[WS2303-024-01, 29.0
Lab: huchunjie
Not signed
STAR® SW 16.20

**Figure 3**

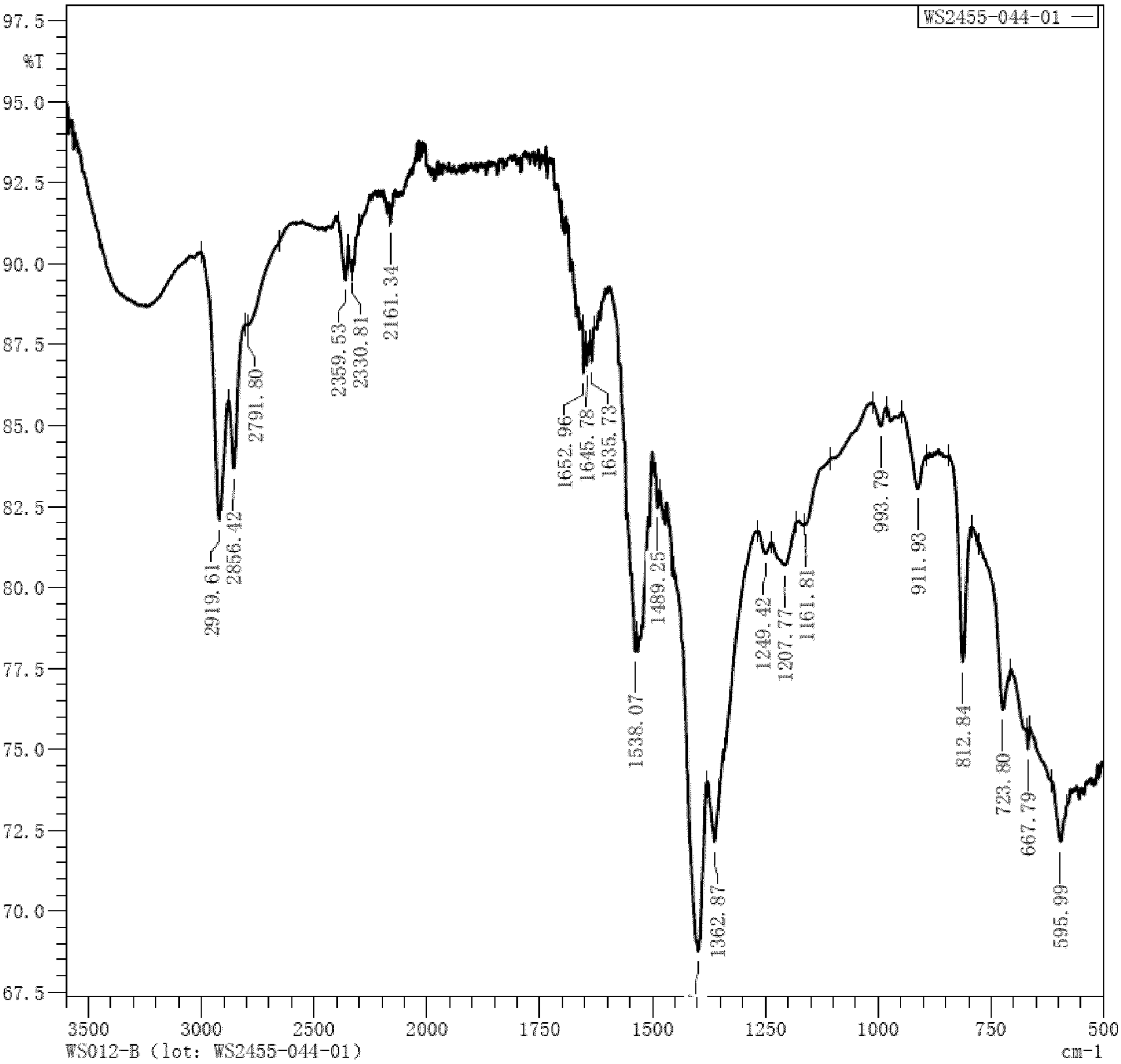
WS2455-044-01
%T
97.5
95.0
92.5
90.0
87.5
85.0
82.5
80.0
77.5
75.0
72.5
70.0
67.5
3500
3000
2500
2000
1750
1500
1250
1000
750
500
cm-1
WS012-B (lot: WS2455-044-01)
2919.61
2856.42
2791.80
2359.53
2330.81
2161.34
1652.96
1645.78
1635.73
1538.07
1489.25
1362.87
1249.42
1207.77
1161.81
993.79
911.93
812.84
723.80
667.79
595.99

Figure 4

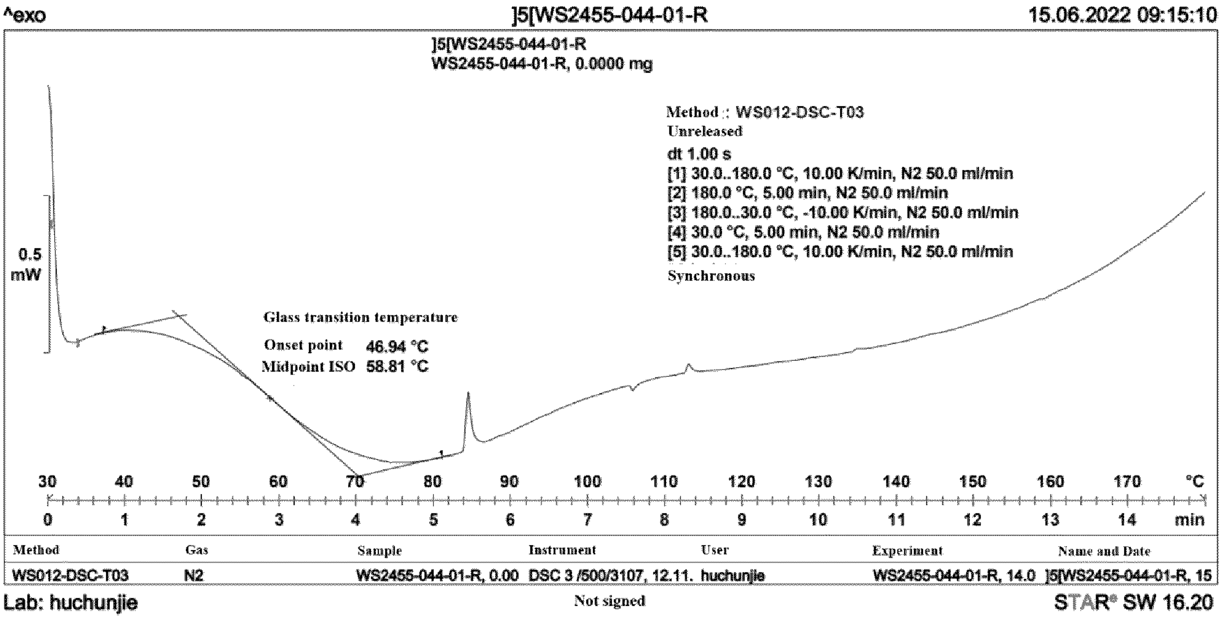


Figure 5

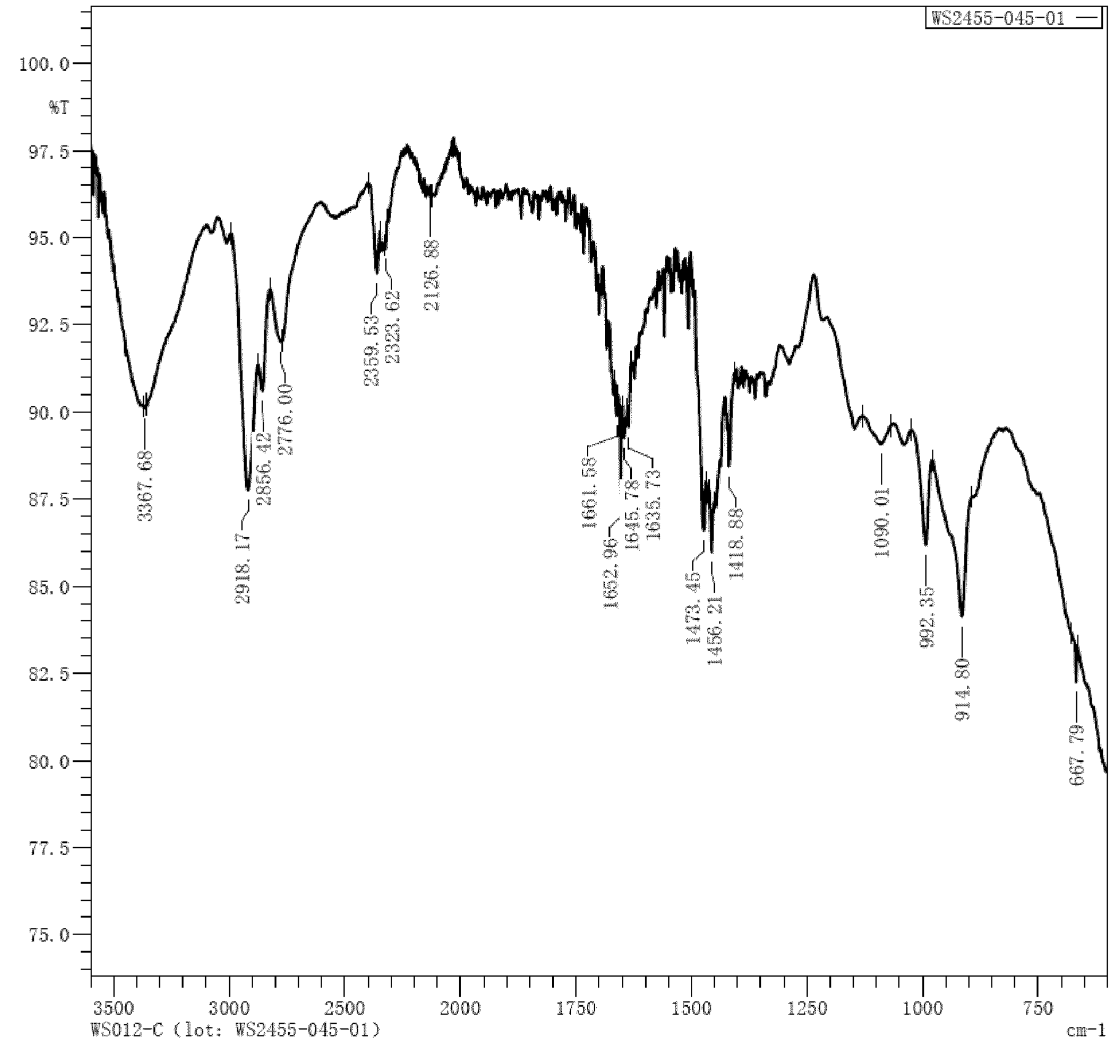


Figure 6

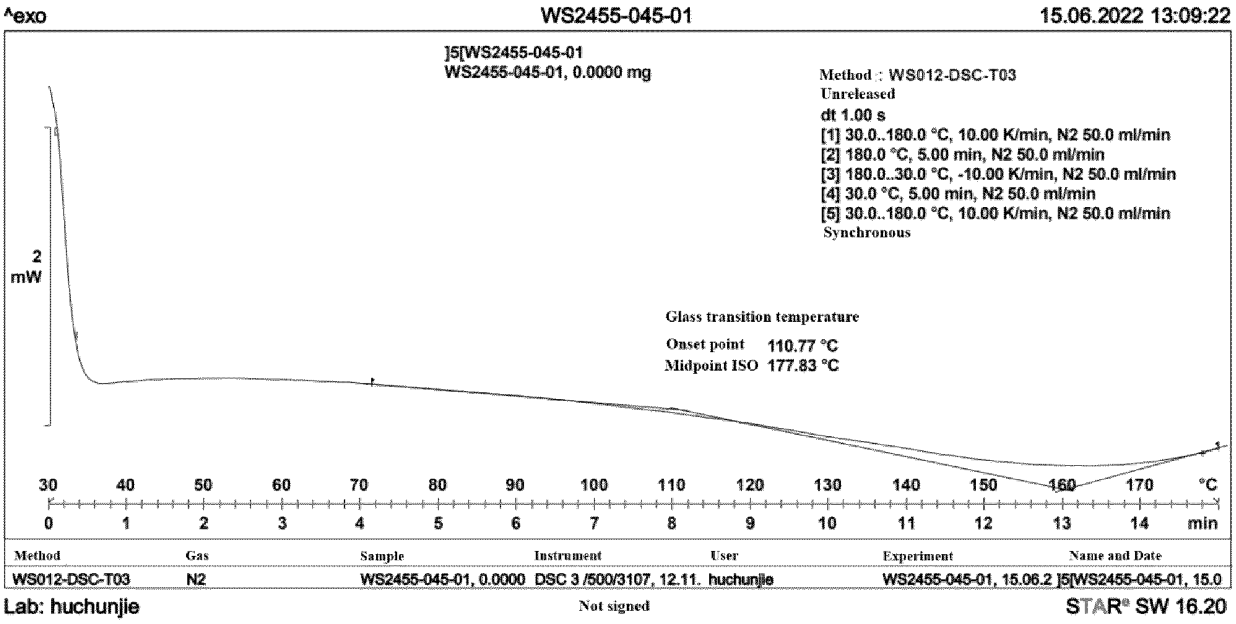

^exo
WS2455-045-01
15.06.2022 13:09:22
]5[WS2455-045-01
WS2455-045-01, 0.0000 mg
Method : WS012-DSC-T03
Unreleased
dt 1.00 s
[1] 30.0..180.0 °C, 10.00 K/min, N2 50.0 ml/min
[2] 180.0 °C, 5.00 min, N2 50.0 ml/min
[3] 180.0..30.0 °C, -10.00 K/min, N2 50.0 ml/min
[4] 30.0 °C, 5.00 min, N2 50.0 ml/min
[5] 30.0..180.0 °C, 10.00 K/min, N2 50.0 ml/min
Synchronous
2
mW
Glass transition temperature
Onset point 110.77 °C
Midpoint ISO 177.83 °C
30 40 50 60 70 80 90 100 110 120 130 140 150 160 170 °C
0 1 2 3 4 5 6 7 8 9 10 11 12 13 14 min
Method
Gas
Sample
Instrument
User
Experiment
Name and Date
WS012-DSC-T03
N2
WS2455-045-01, 0.0000
DSC 3 /500/3107, 12.11.
huchunjie
WS2455-045-01, 15.06.2
]5[WS2455-045-01, 15.0
Lab: huchunjie
Not signed
STAR® SW 16.20


Figure 7

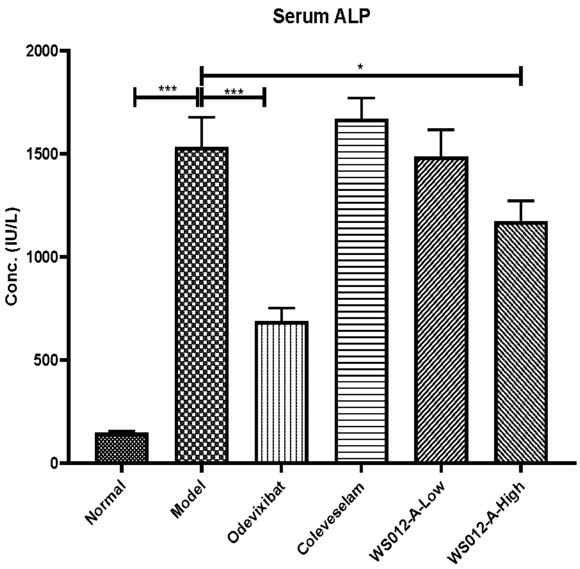


*P<0.05, **P<0.01, ***P<0.001

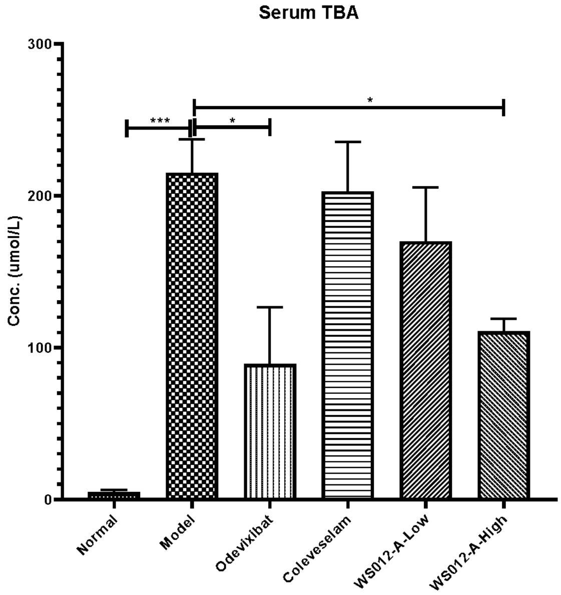


*P<0.05, **P<0.01,***P<0.001

Figure 8A Figure 8B

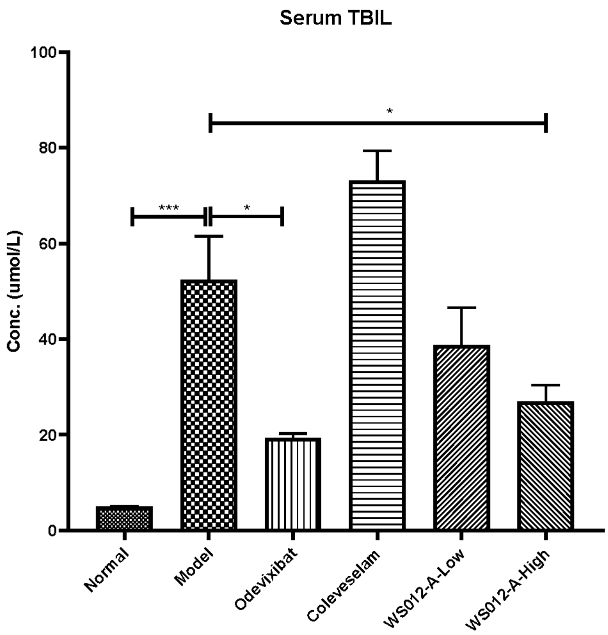


*P<0.05, **P<0.01, ***P<0.001

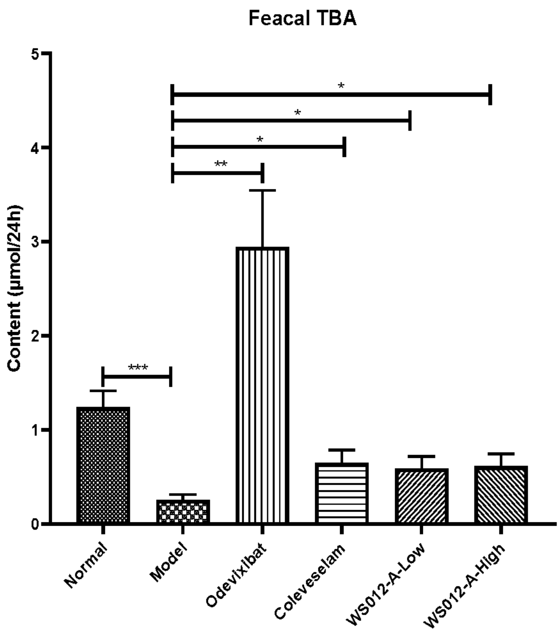


*P<0.05, **P<0.01, ***P<0.001

Figure 8C Figure 8D

**Figure 8**

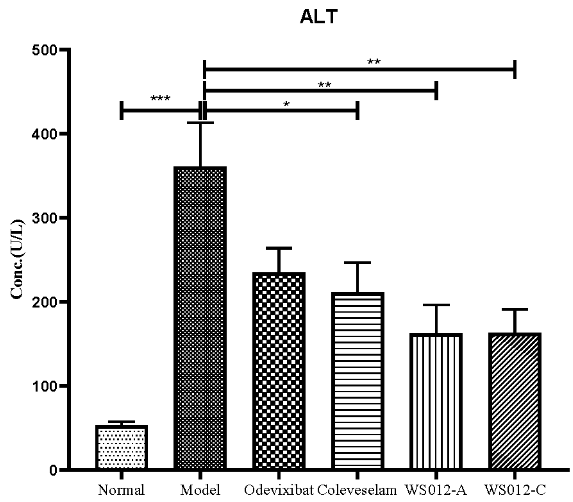


*P<0.05, **P<0.01, ***P<0.001

Figure 9A

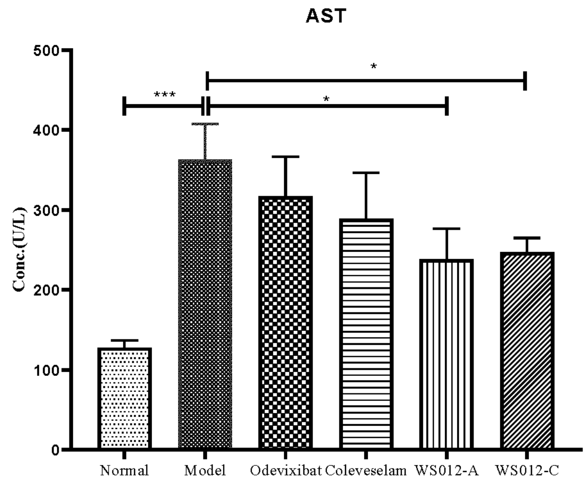


*P<0.05, **P<0.01, ***P<0.001

Figure 9B

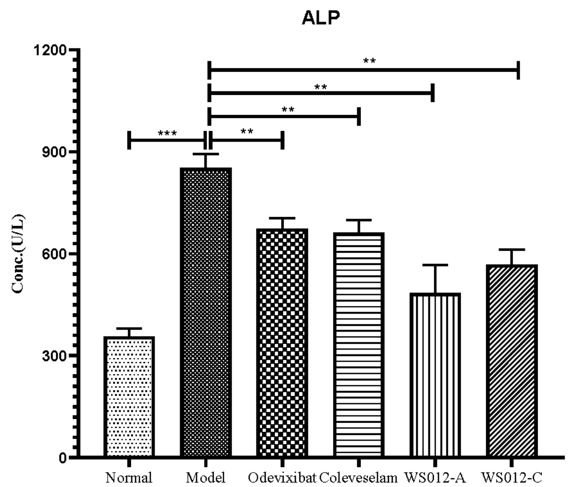


*P<0.05, **P<0.01, ***P<0.001

Figure 9C

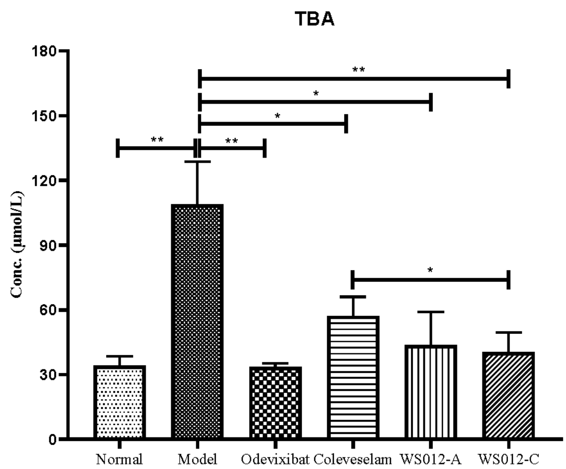


*P<0.05, **P<0.01

Figure 9D

**Figure 9**

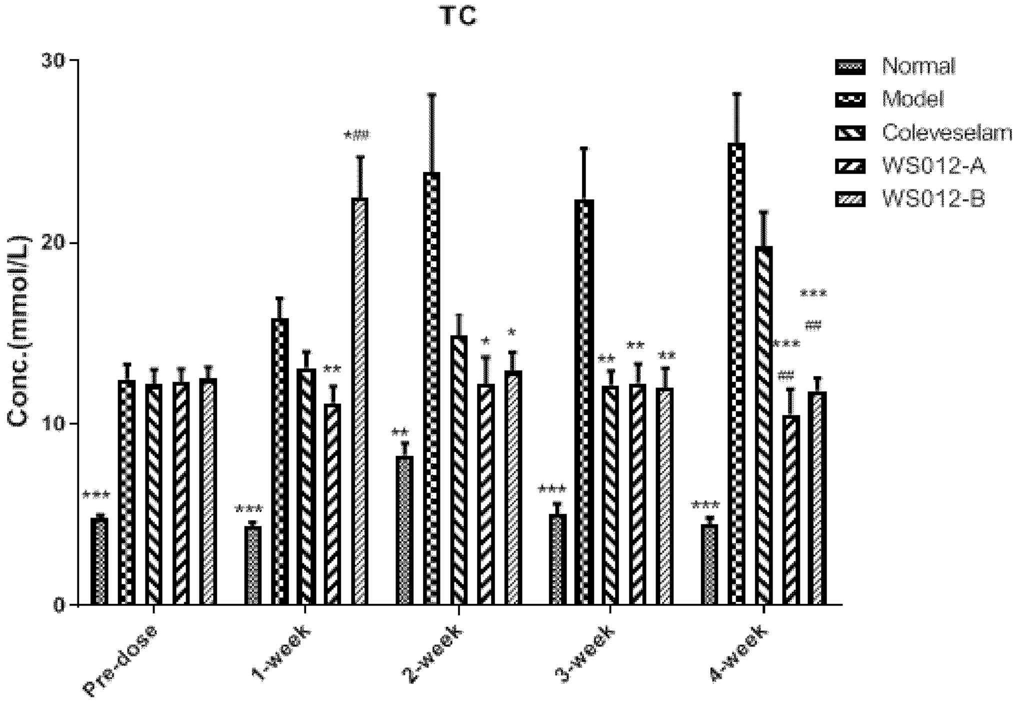


vs Model, *P<0.05, **P<0.01, ***P<0.001; vs Coleveselam, #P<0.05, ##P<0.01

Figure 10A

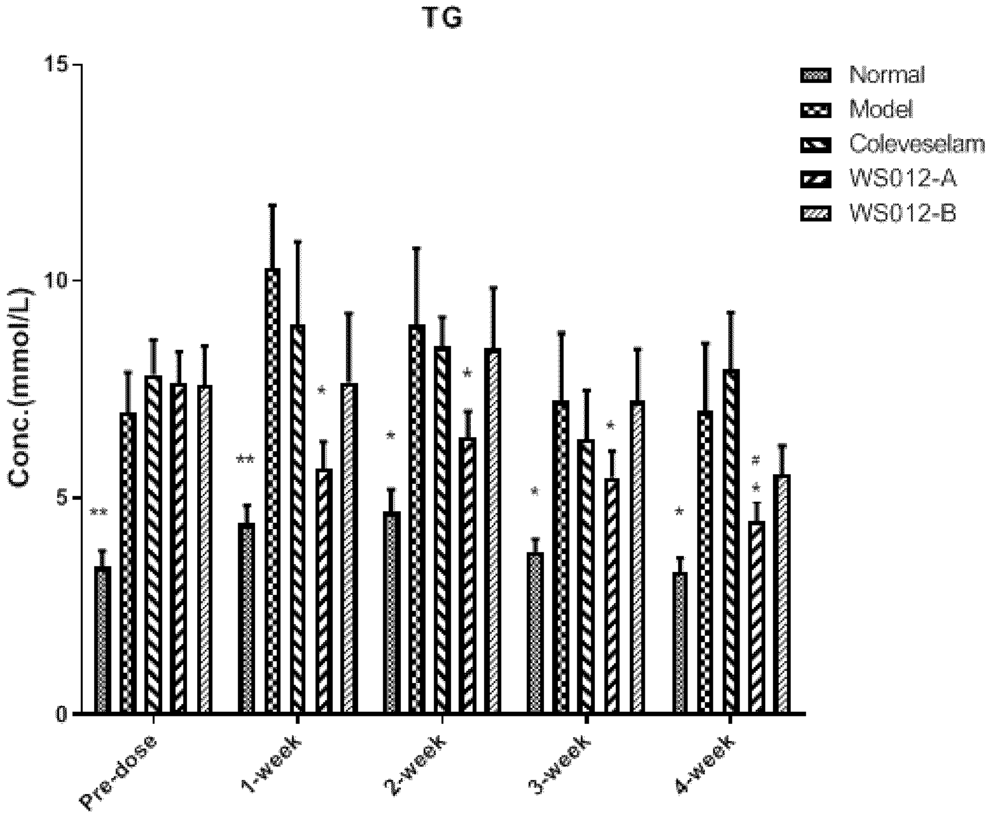


vs Model, *P<0.05, **P<0.01; vs Coleveselam, #P<0.05

Figure 10B

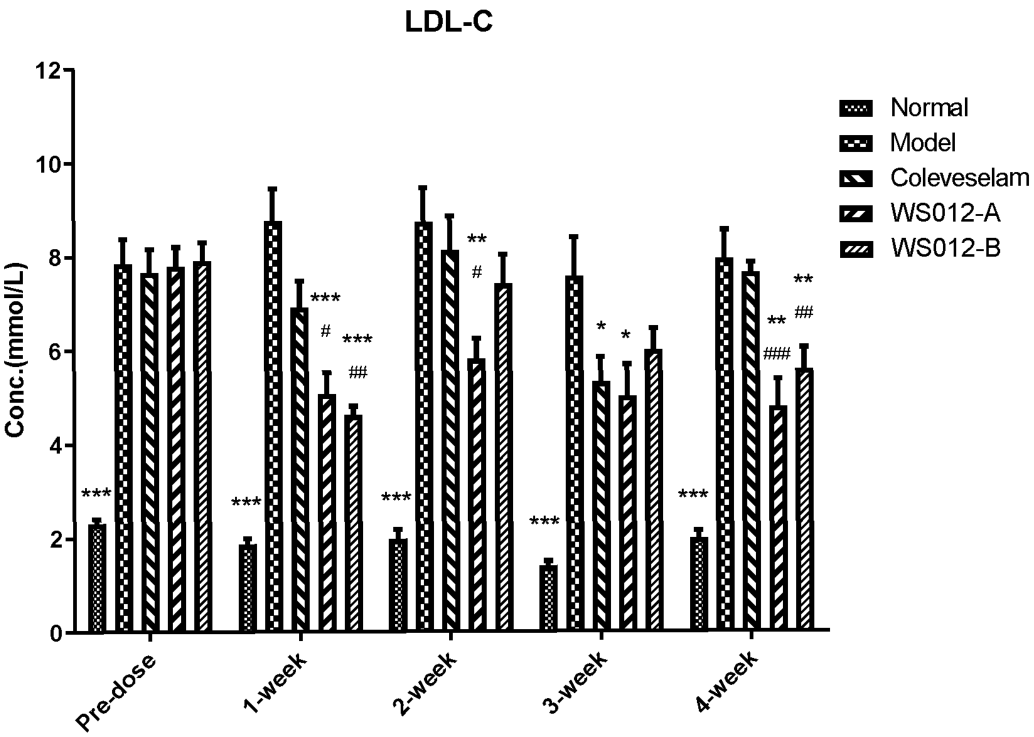


vs Model,*P<0.05,**P<0.01,***P<0.001;vs Coleveselam ,#P<0.05,##P<0.01,###P<0.001

Figure 10C

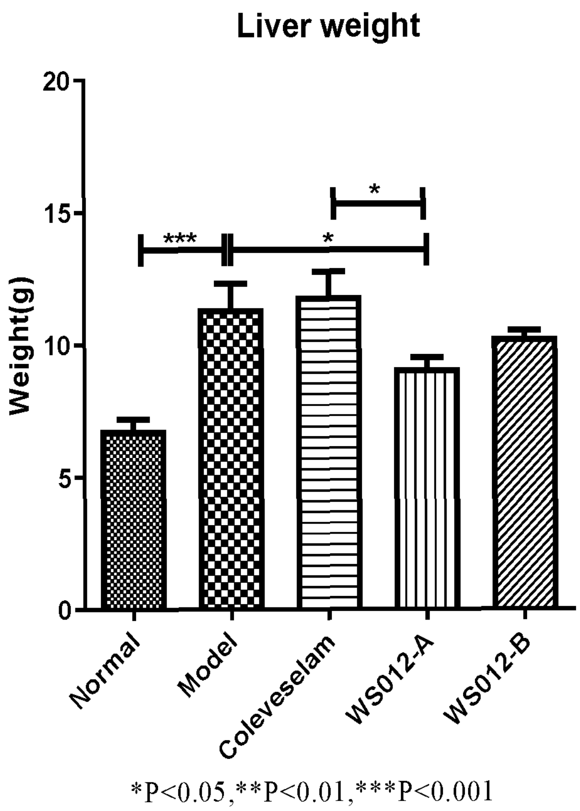


*P<0.05,**P<0.01,***P<0.001

Figure 10D

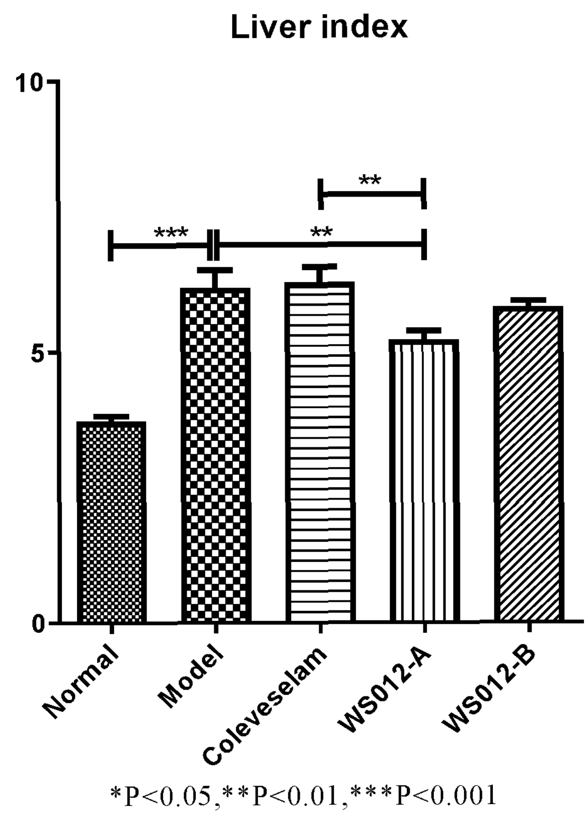


*P<0.05,**P<0.01,***P<0.001

Figure 10E

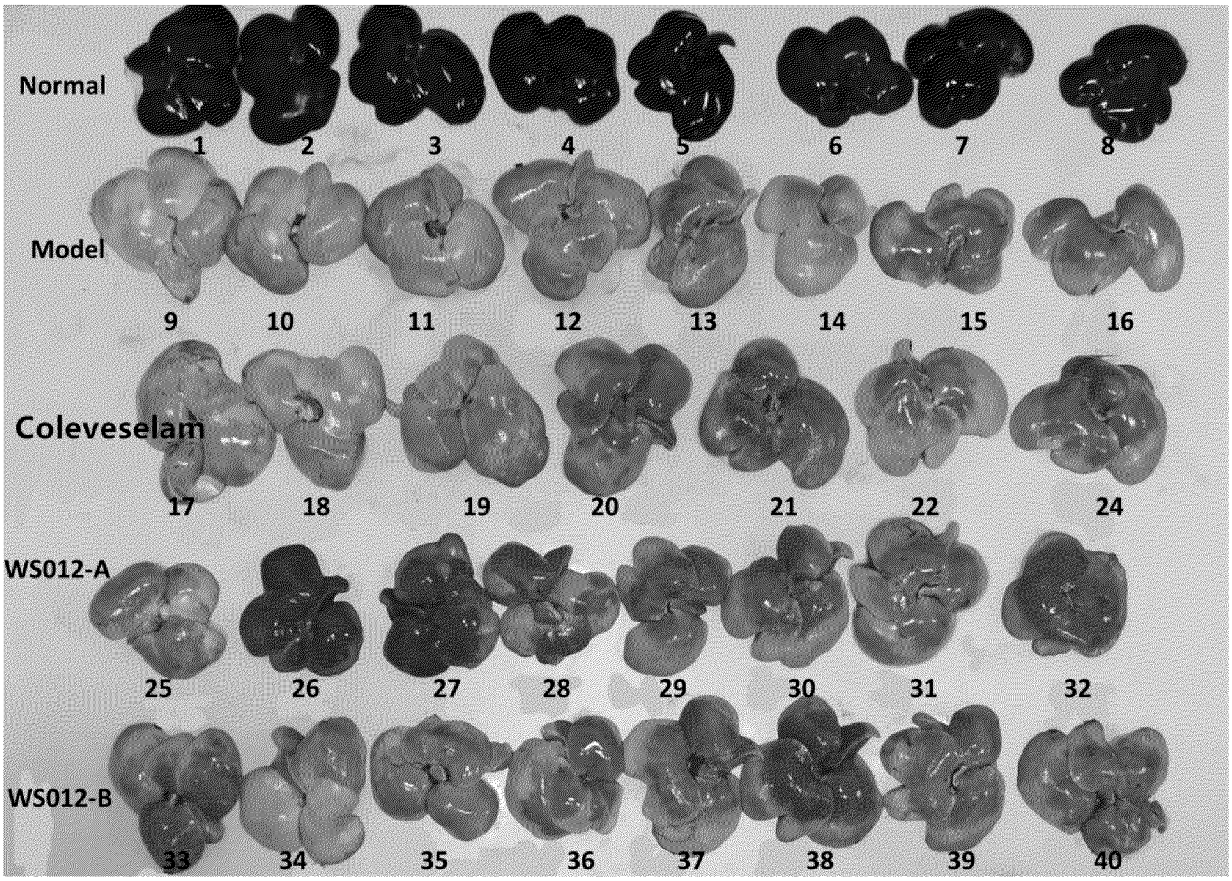


Figure 10F

**Figure 10**

## INTERNATIONAL SEARCH REPORT

International application No.
**PCT/CN2022/113905**

**A. CLASSIFICATION OF SUBJECT MATTER**

A61K 31/785(2006.01)i；C08F 226/02(2006.01)i

According to International Patent Classification (IPC) or to both national classification and IPC

**B. FIELDS SEARCHED**

Minimum documentation searched (classification system followed by classification symbols)

A61K31/-，C08F226/-

Documentation searched other than minimum documentation to the extent that such documents are included in the fields searched

Electronic data base consulted during the international search (name of data base and, where practicable, search terms used)

CNPAT, WPI, CNKI, ISI, STN: 胆汁酸, 二烯丙基胺, 三烯丙基胺, 124-02-7, 102-70-5, 结构式检索, search for structural formula, bile acids, diallylamine, triallylamine

**C. DOCUMENTS CONSIDERED TO BE RELEVANT**

| Category* | Citation of document, with indication, where appropriate, of the relevant passages | Relevant to claim No. |
|---|---|---|
| X | WO 9922721 A2 (GELTEX PHARMACEUTICALS, INC.) 14 May 1999 (1999-05-14) description, page 5 paragraphs 2, 3, embodiment 2 | 1-15 |
| A | CN 102858817 A (RELYPSA, INC.) 02 January 2013 (2013-01-02) entire document | 1-15 |
| A | CN 1056056 A (ROHM AND HAAS COMPANY) 13 November 1991 (1991-11-13) entire document | 1-15 |
| A | CN 102906159 A (RELYPSA, INC.) 30 January 2013 (2013-01-30) entire document | 1-15 |
| A | US 2013156720 A1 (CURRIE MARK G. et al.) 20 June 2013 (2013-06-20) entire document | 1-15 |
| A | JP 2013181130 A (SENKA K.K.) 12 September 2013 (2013-09-12) entire document | 1-12 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 December 2022** | **21 December 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/113905**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **15**
because they relate to subject matter not required to be searched by this Authority, namely:

[1] Claim 15 relates to a method for treating or preventing diseases associated with excessive bile acid, and therefore said claim does not comply with PCT Rule 39.1(iv). A search is performed on the basis of the designation of the subject matter being the use of a drug for treating or preventing diseases associated with excessive bile acid.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/113905**

| Patent document cited in search report | Publication date (day/month/year) | Patent family member(s) | Publication date (day/month/year) |
|---|---|---|---|
| WO 9922721 A2 | 14 May 1999 | US 6365186 B1 | 02 April 2002 |
| | | EP 1028717 A2 | 23 August 2000 |
| | | AU 1364699 A | 24 May 1999 |
| | | CN 1278180 A | 27 December 2000 |
| | | ES 2196625 T3 | 16 December 2003 |
| | | AR 013742 A1 | 10 January 2001 |
| | | NZ 504216 A | 26 November 2002 |
| | | HU 0004279 A2 | 28 March 2002 |
| | | US 2010266526 A1 | 21 October 2010 |
| | | NO 20002172 D0 | 27 April 2000 |
| | | US 2002155091 A1 | 24 October 2002 |
| | | ZA 989673 B | 11 May 1999 |
| | | DE 69812681 D1 | 30 April 2003 |
| | | KR 20010031822 A | 16 April 2001 |
| | | US 6083497 A | 04 July 2000 |
| | | TW 453878 B | 11 September 2001 |
| | | BR 9813961 A | 26 September 2000 |
| | | US 6264938 B1 | 24 July 2001 |
| | | JP 2001521890 A | 13 November 2001 |
| | | US 6248318 B1 | 19 June 2001 |
| | | CA 2309027 A1 | 14 May 1999 |
| | | IL 135858 D0 | 20 May 2001 |
| | | PL 340296 A1 | 29 January 2001 |
| | | AT 235236 T | 15 April 2003 |
| CN 102858817 A | 02 January 2013 | JP 2017048398 A | 09 March 2017 |
| | | JP 2015038225 A | 26 February 2015 |
| | | EP 2539380 A2 | 02 January 2013 |
| | | AU 2011220742 A1 | 13 September 2012 |
| | | ES 2549513 T3 | 28 October 2015 |
| | | JP 2013520561 A | 06 June 2013 |
| | | BR 112012021444 A2 | 31 May 2016 |
| | | CA 2790999 A1 | 01 September 2011 |
| | | US 2016051576 A1 | 25 February 2016 |
| | | AU 2016203432 A1 | 16 June 2016 |
| | | WO 2011106542 A2 | 01 September 2011 |
| | | BR 112014021444 A2 | 22 August 2017 |
| | | US 2013022570 A1 | 24 January 2013 |
| | | CN 105017481 A | 04 November 2015 |
| | | EP 2998329 A2 | 23 March 2016 |
| CN 1056056 A | 13 November 1991 | EP 0459632 A1 | 04 December 1991 |
| | | KR 910019631 A | 19 December 1991 |
| | | AU 7592991 A | 07 November 1991 |
| | | NO 911700 D0 | 30 April 1991 |
| | | CS 126491 A3 | 19 February 1992 |
| | | JP H04298508 A | 22 October 1992 |
| | | HU 911391 D0 | 28 October 1991 |
| | | IL 97973 D0 | 21 June 1992 |
| | | CA 2040996 A1 | 03 November 1991 |
| | | NZ 237993 A | 26 August 1993 |
| | | IE 911405 A1 | 06 November 1991 |

Form PCT/ISA/210 (patent family annex) (January 2015)

**INTERNATIONAL SEARCH REPORT**
**Information on patent family members**

International application No.
**PCT/CN2022/113905**

| Patent document cited in search report | Publication date (day/month/year) | Patent family member(s) | Publication date (day/month/year) |
|---|---|---|---|
| | | FI 912109 A0 | 30 April 1991 |
| | | PT 97549 A | 31 January 1992 |
| | | PL 290121 A1 | 27 January 1992 |
| CN 102906159 A | 30 January 2013 | DE 112011100657 T5 | 28 February 2013 |
| | | GB 201216820 D0 | 07 November 2012 |
| | | US 2017258825 A1 | 14 September 2017 |
| | | CN 107266681 A | 20 October 2017 |
| | | CA 2790901 A1 | 01 September 2011 |
| | | JP 2016130320 A | 21 July 2016 |
| | | AU 2011220748 A1 | 20 September 2012 |
| | | BR 112012021446 A2 | 31 May 2016 |
| | | WO 2011106548 A1 | 01 September 2011 |
| | | RU 2012140432 A | 27 March 2014 |
| | | JP 2017190464 A | 19 October 2017 |
| | | EP 2539389 A1 | 02 January 2013 |
| | | ES 2661079 T3 | 27 March 2018 |
| | | US 2014356316 A1 | 04 December 2014 |
| | | AU 2016200700 A1 | 25 February 2016 |
| | | KR 20120130227 A | 29 November 2012 |
| | | JP 2013520563 A | 06 June 2013 |
| | | RU 2017106587 A | 21 January 2019 |
| | | MX 2012009783 A | 21 November 2012 |
| US 2013156720 A1 | 20 June 2013 | WO 2012027331 A1 | 01 March 2012 |
| JP 2013181130 A | 12 September 2013 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Chinese Pharmacopoeia. 2020, vol. IV, 0402
- **PETER FICKERT**. *The American Journal of Pathology*, August 2007, vol. 171 (2), 525-536
- **YOSHIJI OHTA et al.** *Toxicology*, 1999, vol. 139, 265-275